(19) Europäisches Patentamt
European Patent Office
Office européen des brevets

(11) **EP 1 639 125 B1**

(12) **EUROPEAN PATENT SPECIFICATION**

(45) Date of publication and mention
of the grant of the patent:
**16.09.2009 Bulletin 2009/38**

(51) Int Cl.:
*C12Q 1/48* (2006.01)      *G01N 33/58* (2006.01)
*G01N 33/92* (2006.01)      *C12Q 1/60* (2006.01)
*C12Q 1/61* (2006.01)

(21) Application number: **04816755.5**

(22) Date of filing: **25.06.2004**

(86) International application number:
**PCT/US2004/020542**

(87) International publication number:
**WO 2005/043110 (12.05.2005 Gazette 2005/19)**

(54) **FLUORESCENCE ASSAY FOR MTP ACTIVITY**

FLUORESZENZ-ASSAY FÜR MTP-AKTIVITÄT

ESSAI BIOLOGIQUE A FLUORESCENCE POUR L'ACTIVITE DES MTP

(84) Designated Contracting States:
**AT BE BG CH CY CZ DE DK EE ES FI FR GB GR HU IE IT LI LU MC NL PL PT RO SE SI SK TR**

(30) Priority: **27.06.2003 US 483321 P**

(43) Date of publication of application:
**29.03.2006 Bulletin 2006/13**

(73) Proprietor: **The Research Foundation of State University of New York**
**Albany, NY 12207 (US)**

(72) Inventors:
• **HUSSAIN, M., Mahmood**
**Woodbury, NY 11797 (US)**
• **ATHAR, Humra**
**Brookline, MA 02445 (US)**
• **IQBAL, Jahangir**
**Jersey City, NJ 07306 (US)**

(74) Representative: **De Clercq, Ann G. Y. et al**
**De Clercq, Brants & Partners c.v.**
**Edgard Gevaertdreef 10a**
**9830 Sint-Martens-Latem (BE)**

(56) References cited:
EP-A- 0 584 446          WO-A-01/05762
WO-A-01/30354           WO-A-95/22621
US-A- 5 770 355

• JIN FU-YOU ET AL: "Niacin accelerates intracellular apoB degradation by inhibiting triacylglycerol synthesis in human hepatoblastoma (HepG2) cells" ARTERIOSCLEROSIS THROMBOSIS AND VASCULAR BIOLOGY, vol. 19, no. 4, April 1999 (1999-04), pages 1051-1059, XP002389128 ISSN: 1079-5642
• PERLEMUTER GABRIEL ET AL: "Hepatitis C virus core protein inhibits microsomal triglyceride transfer protein activity and very low density lipoprotein secretion: A model of viral-related steatosis" FASEB JOURNAL, vol. 16, no. 2, February 2002 (2002-02), pages 185-194, XP002389129 ISSN: 0892-6638
• TAGUCHI HIROYUKI ET AL: "Dietary diacylglycerol suppresses high fat diet-induced hepatic fat accumulation and microsomal triacylglycerol transfer protein activity in rats." JOURNAL OF NUTRITIONAL BIOCHEMISTRY, vol. 13, no. 11, November 2002 (2002-11), pages 678-683, XP002389130 ISSN: 0955-2863
• WETTERAU J R ET AL: "Protein disulfide isomerase appears necessary to maintain the catalytically active structure of the microsomal triglyceride transfer protein" BIOCHEMISTRY, AMERICAN CHEMICAL SOCIETY. EASTON, PA, US, vol. 30, 1991, pages 9728-9735, XP002231796 ISSN: 0006-2960
• ATZEL AMY ET AL: "Mechanism of microsomal triglyceride transfer protein catalyzed lipid transport" BIOCHEMISTRY, vol. 32, no. 39, 1993, pages 10444-10450, XP002389131 ISSN: 0006-2960

EP 1 639 125 B1

**(Cont. next page)**

- ATZEL AMY ET AL: "Identification of Two Classes of Lipid Molecule Binding Sites on the Microsomal Triglyceride Transfer Protein" BIOCHEMISTRY, vol. 33, no. 51, 1994, pages 15382-15388, XP002389132 ISSN: 0006-2960
- FUNATSU T ET AL: "PROLONGED INHIBITION OF CHOLESTEROL SYNTHESIS BY ATORVASTATIN INHIBITS APO B-100 AND TRIGLYCERIDE SECRETION FROM HEPG2 CELLS" ATHEROSCLEROSIS, AMSTERDAM, NL, vol. 157, no. 1, 2001, pages 107-115, XP001075042 ISSN: 0021-9150
- FISHER EDWARD A ET AL: "The triple threat to nascent apolipoprotein B: Evidence for multiple, distinct degradative pathways" JOURNAL OF BIOLOGICAL CHEMISTRY, vol. 276, no. 30, 27 July 2001 (2001-07-27), pages 27855-27863, XP002389133 ISSN: 0021-9258
- PHILLIPS CATHERINE ET AL: "Intestinal rather than hepatic microsomal triglyceride transfer protein as a cause of postprandial dyslipidemia in diabetes" METABOLISM CLINICAL AND EXPERIMENTAL, vol. 51, no. 7, July 2002 (2002-07), pages 847-852, XP002389134 ISSN: 0026-0495
- HIGASHI YUSUKE ET AL: "Transmembrane lipid transfer is crucial for providing neutral lipids during very low density lipoprotein assembly in endoplasmic reticulum." JOURNAL OF BIOLOGICAL CHEMISTRY, vol. 278, no. 24, 13 June 2003 (2003-06-13), pages 21450-21458, XP002389141 ISSN: 0021-9258

**Description**

**BACKGROUND OF THE INVENTION**

Microsomal triglyceride transfer protein (MTP) is a dedicated chaperone that is required for the assembly of apolipoprotein B (apoB) lipoproteins [for reviews, see refs. (1-6)]. It is believed that MTP transfers lipids to nascent apoB in the endoplasmic reticulum and renders it secretion-competent by forming primordial lipoprotein particles [for reviews, see refs. (1-9)]. The importance of MTP's lipid transfer activity in apoB secretion has been established by three independent approaches. First, mutations in MTP have been correlated with the absence of apoB lipoproteins in abetalipoproteinemia (10, 11). Second, antagonists that inhibit MTP's lipid transfer activity *in vitro* have been shown to decrease apoB secretion *in vivo* (12-14). Third, the coexpression of MTP with apoB has been demonstrated to facilitate apoB secretion in cells that do not express apoB and MTP (15, 16). In addition to its lipid transfer activity, MTP is known to physically interact with apoB (1, 2). Recently, MTP has been implicated in the import of triacylglycerols (TAGs) from cytosol to the lumen of the endoplasmic reticulum (17-19). Thus, it appears that MTP is a multifunctional protein (2) that plays a crucial role in the transport of TAG within intracellular organelles and in its secretion out of the cells.

MTP was identified and purified by Wetterau and Zilversmit (20, 21) based on a radioisotope assay. In this assay, radiolabeled TAGs are incorporated into donor vesicles consisting of phosphatidylcholine (PC) and cardiolipin. These vesicles are incubated with acceptor vesicles in the presence of MTP. After 1-3 h of incubation, the cardiolipin-containing donor vesicles are allowed to bind to DE52 and removed by centrifugation. Radioactivity remaining in the supernatant is quantified by scintillation counting. This procedure is labor-intensive and time-consuming. Negatively charged lipids, such as cardiolipin, are known to inhibit the lipid transfer activity of MTP (22). Because of the multiple steps involved in this procedure, it is difficult to automate. Thus, it would be advantageous to have a simple, one-step procedure to measure MTP activity. Such a procedure would be useful, e.g., in identifying compounds that partially inhibit MTP activity and therefore decrease lipoprotein assembly and secretion. The identified compounds are highly desirable as drugs for decreasing plasma cholesterol and triglyceride levels in cells.

Several pharmaceutical companies have identified antagonists that inhibit MTP activity (12-14, 23). A general approach taken by these companies is to identify compounds that decrease apoB secretion by HepG2 cells and then to determine their ability to inhibit MTP activity (3, 23). The primary screening involving the inhibition of apoB secretion might have been attributable to the difficulties involved in evaluating large numbers of compounds for MTP inhibition using a multi-step radioactive assay (20, 21). This two-step screening has resulted in the identification of compounds that inhibit MTP activity and decrease apoB secretion. Surprisingly, different compounds identified by various companies are structurally very similar (23). Unfortunately, these compounds cause significant accumulation of TAG in cells raising the possibility that the selected compounds are potent inhibitors of apoB secretion. Ideally, compounds that inhibit MTP activity but that have a partial effect on lipoprotein secretion are sought after. For this purpose, it is be desirable to develop methods that can screen compounds primarily for their ability to inhibit the lipid transfer activity of MTP. The present invention provides simple, rapid, and sensitive methods to assay MTP activity that are amenable to automation and high-throughput screening.

**SUMMARY OF THE INVENTION**

The present invention provides methods and compositions for measuring MTP activity as defined in the claims. In one aspect of the invention, there is provided a method of measuring levels of MTP comprising the steps of:

(a) preparing phosphatidylcholine (PC)donor vesicles having a fluorescence-labeled triglyceride lipid incorporated therein;
(b) preparing phosphatidylcholine (PC) acceptor vesicles,
(c) incubating either a cellular homogenate containing MTP or isolated MTP with the acceptor vesicles and the labeled donor vesicles for a time and under conditions sufficient to allow binding of the fluorescence-labeled triglyceride lipid with MTP during transfer of the labeled lipid from donor to acceptor vesicles; wherein the final concentration of phosphatidylcholine of the donor vesicles ranges from $6\mu M$ to $18\mu M$ and the final concentration of fluorescence-labeled triglyceride lipid ranges from $0.21\,\mu M$ to $0.6\mu M$, and wherein the final concentration of phospholipid phosphatidylcholine of the acceptor vesicles ranges from $42\mu M$ to $102\mu M$, (d) measuring fluorescence of the fluorescence-labeled triglyceride lipid bound to the MTP, wherein the vesicles are stabilized by the addition of BSA to the reaction mixture in a concentration of 1mg/ml.

The cellular homogenates may comprise liver cells, intestinal cells, heart cells or any other cells that express MTP including but not limited to cells of animals (including humans), insects and microorganisms.

Fluorescence-labeled lipids for use in the present invention include but are not limited to triglycerides, cholesterol esters (CE) or phospholipids. An example of a fluorescence-labeled triacylglycerol is 1, 2 dioleoyl 3-NBD glycerol (NBD-TAG). Vesicles are preferably small unilamellar vesicles, multi-lamellar vesicles, apoB-lipoprotein vesicles, other lipoproteins, or phosphatidylcholine (PC) vesicles.

The present invention also provides a method for identifying compounds that modulate the lipid transfer activity of MTP. The method comprises the steps of:

(a) incorporating a fluorescenoe-labeled triglyceride lipid into phosphatidylcholine (PC) donor vesicles;

(b) preparing phosphatidylcholine (PC) acceptor vesicles;

(c) mixing an aliquot of acceptors vesicles and the labeled donor vesicles with a test compound, the test compound being a known or unknown modulator of MTP;

(d) adding a cellular homogenate containing MTP or isolated MTP to the mixture containing donor vesicles, acceptor vesicles, and test compound;

(e) incubating a first aliquot of acceptors vesicles, labeled donor vesicles, test compound and MTP for a time and under conditions sufficient to allow binding of the fluoresoence-labeled triglyceride lipid with MTP during transfer of the labeled lipid from donor to acceptor vesicles; wherein the concentration of phosphatidylcholine of the donor vesicles ranges from $6\mu M$ to $18\,\mu M$ and the concentration of fluorescence-labeled triglyceride lipid ranges from $0.21\mu M$ to $0.6\mu M$, and wherein the concentration of phospholipid phosphatidylcholine of the acceptor vesicles ranges from $42\mu M$ to $102\mu M$, wherein the vesicles are stabilized by the addition of 1mg/ml BSA to the reaction mixture;

(f) incubating a second aliquot of acceptor vesicles, labeled donor vesicles and MTP for a time and under conditions sufficient to allow binding of the fluorescence-labeled triglyceride lipid with MTP during transfer of the labeled lipid from donor to acceptor vesicles; wherein the concentration of phospholipid phosphatidylcholine of the donor vesicles ranges from $6\mu M$ to $18\mu M$ and the concentration of fluorescence-labeled triglyceride lipid ranges from $0.21\mu M$ to $0.6\mu M$ , and wherein the concentration of phospholipid phosphatidylcholine of the acceptor vesicles ranges from $42\mu M$ to $102\mu M$, wherein the vesicles are stabilized by the addition of 1mg/ml BSA to the reaction mixture;

(g) measuring fluorescence of the fluorescence-labeled triglyceride lipid bound to MTP obtained in steps (e) and (f); and

(h) correlating an increase in fluorescence of the fluorescence-labeled triglycerides lipids bound to MTP obtained in step (e) when compared to the fluorescence of the fluorescence-labeled triglyceride lipid bound to MTP obtained in step (f) with identification of a compound which increases lipid transfer activity of MTP, while correlating a decrease in fluorescence of the fluorescence-labeled triglyceride lipid bound to MTP obtained in step (e) when compared to the fluorescence of the fluorescence-labeled triglyceride lipid bound to MTP obtained in step (f) with identification of a compound which decreases lipid transfer activity of MTP.

The present invention also provides a mixture for measuring the lipid transfer activity of MTP. The mixture comprises

- phosphatidylcholine (PC) acceptor vesicles,
- phosphatidylcholine (PC) donor vesicles having a fluorescence-labeled triglyceride lipid incorporated therein, and
- BSA,

wherein the concentration of phosphatidylcholine of the donor vesicles ranges from $6\mu M$ to $18\mu M$ and the concentration of fluoresoenoe-labeled triglyceride lipid ranges from $0.21\mu M$ to $0.6\mu M$, and
wherein the concentration of phosphatidylcholine of the acceptor vesicles ranges from $42\mu M$ to $102\mu M$ and wherein the concentration of BSA is 1mg/ml.

Preferably, the fluorescence-labeled donor vesicles of the mixture comprised of a triglyceride, a cholesterol ester, or a phospholipid. Vesicles contained in the mixture may be small unilamellar vesicles, multi-lamellar vesicles, apoB-lipoprotein vesicles, or phosphatidylcholine (PC) vesicles. The vesicles contained in the mixture are preferably admixed with an appropriate buffer.

## BRIEF DESCRIPTION OF THE DRAWINGS

Figures 1A-1D graphically depict the effect of different amounts of donor and acceptor vesicles on the transfer of triacylglycerol (TAG) by microsomal triglyceride transfer protein (MTP). Line graphs and error bars represent means $\pm$ SD (n=3).

Figures 2A-2C graphically depict the effects of time, temperature and NaCl on TAG transfer activity of MTP. Line graphs and error bars represent means $\pm$ SD.

Figures 3A-3C graphically illustrate the specificity of TAG transfer activity.

Figures 4A and 4B are graphs depicting the role of acceptor vesicles in lipid transfer by MTP. Line graphs and error bars represent means $\pm$ SD.

Figures 5A-5I graphically illustrate transfer of various lipids in the presence of different acceptors. Line graphs and error bars represent means $\pm$ SD.

Figures 6A and 6B are graphs comparing two methods to measure MTP activity in cell homogenates. Line graphs and error bars represent means $\pm$ SD.

Figures 7A and 7B graphically depict inhibition of MTP activity by BMS200150. Line graphs and error bars represent means $\pm$ SD.

Figure 8 is a schematic of a unilamellar vesicle with a labeled triglyceride, NBD-TAG, embedded in the bilipid membrane.

## DETAILED DESCRIPTION OF THE INVENTION

MTP activity is classically measured by incubating purified MTP or cellular homogenates with donor vesicles containing radio labeled lipids for 1-3 h, precipitating the donor vesicles, and measuring the radioactivity transferred to acceptor vesicles.

In accordance with the present invention, a new, simple, rapid, and sensitive fluorescence assay for MTP is provided. In this assay, isolated and/or purified MTP or cellular homogenates are incubated with donor vesicles containing quenched fluorescence-labeled lipids (e.g., triglycerides, cholesterol esters, and/or phospholipids) and different types of acceptor vesicles. The cellular homogenate may be made from any cells that express MTP. Preferably, the cells are animal liver cells, intestinal cells or heart cells. Preferably, the animal cells are from a mammal such as a rat, mouse, monkey, or human. Cellular homogenates may also comprise cells from insects or microorganisms.

A fluorescence-labeled lipid may include but is not limited to a triglyceride, a cholesterol ester (CE) or a phospholipid. An example of a triglyceride for use in the present invention is triacylglycerol (TAG).

Examples of different types of fluorescence compounds which may be used for labeling the lipids include but are not limited to 7-nitrobenz-2-oxa-1,3-diazole (NBD), pyrene, or bodipy. Methods of labeling lipids with such fluorescent compounds are well known in the art and prepared fluorescently labeled lipids are also readily available. For use in the present invention, a lipid may contain at least one fluoresecent label at any position. For example, a triacylglycerol may contain at least one NBD at any position while having fatty acids at other positions.

Examples of pyrene-labeled lipids include the following which are available from Molecular Probes, Inc (Eugene, OR) and listed in their on-line catalog as follows:

**B-3782**
1,2-bis-(1-pyrenedecanoyl)-*sn*-glycero-3-phosphocholine

**C-212**
cholesteryl 1-pyrenebutyrate

**D-6562**
1,2-dioleoyl-3-(1-pyrenedodecanoyl)-*rac*-glycerol

**H-361**
1-hexadecanoyl-2-(1-pyrenedecanoyl)-*sn*-glycero-3-phosphocholine ($\beta$-py-$C_{10}$-HPC)

**H-3784**
1-hexadecanoyl-2-(1-pyrenedecanoyl)-*sn*-glycero-3-phosphoethanolamine ($\beta$-py-$C_{10}$-HPE)

**H-3809**
1-hexadecanoyl-2-(1-pyrenedecanoyl)-*sn*-glycero-3-phosphoglycerol, ammonium salt ($\beta$-py-$C_{10}$-PG)

**H-3810**
1-hexadecanoyl-2-(1-pyrenedecanoyl)-*sn*-glycero-3-phosphomethanol, sodium salt ($\beta$-py-$C_{10}$-HPM)

**H-3818**
1-hexadecanoyl-2-(1-pyrenehexanoyl)-*sn*-glycero-3-phosphocholine ($\beta$-py-$C_6$-HPC)

**P-58**
*N*-(1-pyrenesulfonyl)-1,2-dihexadecanoyl-*sn*-glycero-3-phosphoethanolamine, triethylammonium salt (pyS DHPE)

Examples of BODIPY-labeled lipids include the following which are available from Molecular Probes, Inc. and listed in their on-line catalog as follows:

**B-7701**
1,2-bis-(4,4-difluoro-5,7-dimethyl-4-bora-3a,4a-diaza-*s*-indacene-3-undecanoyl)-*sn*-glycero-3-phosphocholine (bis-BODIPY® FL $C_{11}$-PC)

**B-3794**

2-(5-butyl-4,4-difluoro-4-bora-3a,4a-diaza-*s*-indacene-3-nonanoyl)-1-hexadecanoyl-*sn*-glycero-3-phosphocholine (β-C$_4$-BODIPY® 500/510 C$_9$-HPC)

**C-3927**

cholesteryl 4,4-difluoro-5,7-dimethyl-4-bora-3a,4a-diaza-*s*-indacene-3-dodecanoate (cholesteryl BODIPY® FL C$_{12}$)

**C-12680**

cholesteryl 4,4-difluoro-5-(4-methoxyphenyl)-4-bora-3a,4a-diaza-*s*-indacene-3-undecanoate (cholesteryl BODIPY® 542/563 C$_{11}$)

**C-12681**

cholesteryl 4,4-difluoro-5-(2-pyrrolyl)-4-bora-3a,4a-diaza-*s*-indacene-3-undecanoate (cholesteryl BODIPY® 576/589 C$_{11}$)

**D-3792**

2-(4,4-difluoro-5,7-dimethyl-4-bora-3a,4a-diaza-*s*-indacene-3-dodecanoyl)-1-hexadecanoyl-*sn*-glycero-3-phosphocholine (β-BODIPY® FL C$_{12}$-HPC)

**D-3803**

2-(4,4-difluoro-5,7-dimethyl-4-bora-3a,4a-diaza-*s*-indacene-3-pentanoyl)-1-hexadecanoyl-*sn*-glycero-3-phosphocholine (β-BODIPY® FL C$_5$-HPC)

**D-3800**

*N*-(4,4-difluoro-5,7-dimethyl-4-bora-3a,4a-diaza-*s*-indacene-3-propionyl)-1,2-dihexadecanoyl-*sn*-glycero-3-phosphoethanolamine, triethylammonium salt (BODIPY® FL DHPE)

**D-12656**

*N*-(4,4-difluoro-5,7-dimethyl-4-bora-3a,4a-diaza-*s*-indacene-3-propionyl)-1,2-dihexanoyl-*sn*-glycero-3-phosphoethanolamine, triethylammonium salt (BODIPY® FL dicaproyl PE)

**D-3813**

2-(4,4-difluoro-5,7-diphenyl-4-bora-3a,4a-diaza-*s*-indacene-3-dodecanoyl)-1-hexadecanoyl-*sn*-glycero-3-phosphoethanolamine (β-BODIPY® 530/550 C$_{12}$-HPE)

**D-3815**

2-(4,4-difluoro-5,7-diphenyl-4-bora-3a,4a-diaza-*s*-indacene-3-pentanoyl)-1-hexadecanoyl-*sn*-glycero-3-phosphocholine (β-BODIPY® 530/550 C$_5$-HPC)

**D-3799**

*N*-(4,4-difluoro-5,7-diphenyl-4-bora-3a,4a-diaza-*s*-indacene-3-propionyl)-1,2-dihexadecanoyl-*sn*-glycero-3-phosphoethanolamine, triethylammonium salt (BODIPY® 530/550 DHPE)

**D-3793**

2-(4,4-difluoro-5-methyl-4-bora-3a,4a-diaza-*s*-indacene-3-dodecanoyl)-1-hexadecanoyl-*sn*-glycero-3-phosphocholine (β-BODIPY® 500/510 C$_{12}$-HPC)

**D-3795**

2-(4,4-difluoro-5-octyl-4-bora-3a,4a-diaza-*s*-indacene-3-pentanoyl)-1-hexadecanoyl-*sn*-glycero-3-phosphocholine (β-C$_8$-BODIPY® 500/510 C$_5$-HPC)

**D-3806**

2-(4,4-difluoro-5-(4-phenyl-1,3-butadienyl)-4-bora-3a,4a-diaza-*s*-indaceno-3-pentanoyl)-1-hexadecanoyl-*sn*-glycero-3-phosphocholine (β-BODIPY® 581/591 C$_5$-HPC)

NBD labeled lipids may also be custom synthesized by Molecular Probes, Inc., e.g., D-16408: Custom synthesis of 1,3-diolein, 2-NBD-X ester or B-1800: Custom synthesis of NBD-labeled cholesterol oleate (cholesterol ester).

In addition, the following compounds are available from Molecular Probes, Inc. and do not required custom synthesis:

N-00360 (NBD-PE) N-(7-nitrobenz-2-oxa-1,3-diazol-4-yl)-1,2-dihexadecanoyl-*sn*-glycerol-3-phosphoethanolamine, triethylammonium;

N-03786 (NBD C6-HPC) 2-(6-(7-nitrobenz-2-oxa-1,3-diazol-4-yl)amino) hexanoyl-1-hexadecanoyl-*sn*-glycero-3-phosphocholine; and

N-03787 (NBD C12-HPC) 2-(12-(7-nitrobenz-2-oxa-1,3-diazol-4-yl)amino dodecanoyl-1-hexadecanoyl-*sn*-glycero-3-phosphocholine.

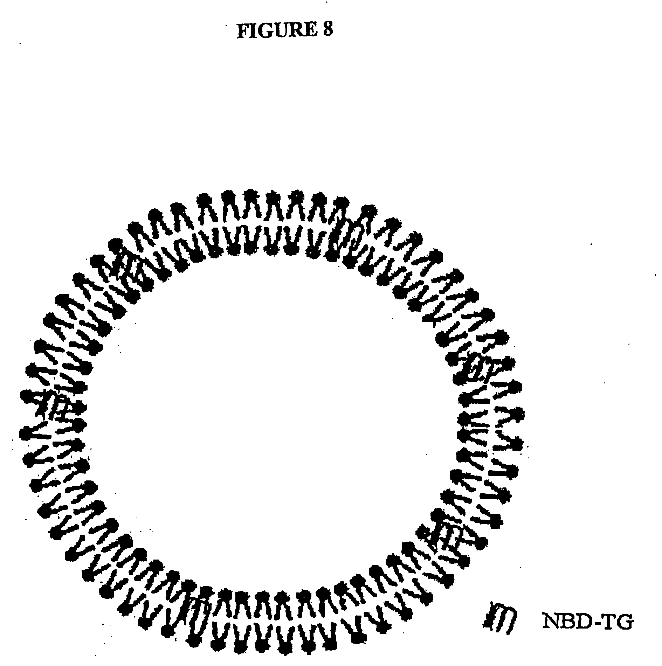

Examples of vesicles include small unilamellar vesicles, multi-lamellar vesicles, apoB-lipoprotein vesicles or phosphatidylcholine (PC) vesicles. As used herein and consistent with the understanding of those skilled in the art, "unilamellar vesicles" mean vesicles (liposomes) having one phospholipid bilayer. "Multi-lamellar vesicles" mean vesicles (liposomes) having several phospholipid bilayers. Figure 8 schematically depicts a unilamellar vesicle with a labeled triglyceride, NBD-TG, embedded in the bilipid membrane. Methods for making acceptor vesicles are known. *See e.g.,* refs. 20, 21, and 30-32. Methods of making donor vesicles are also well known, *see e.g.*, refs. 20, 21, 30-32. In accordance with the present invention, when making the subject donor vesicles, cardiolipin may be omitted and radiolabeled TAGs replaced with fluorescence-labeled TAGs.

Both donor and acceptor vesicles are preferably admixed with an appropriate buffer such as e.g., Tris-HCl, pH at around 7.4. The donor and acceptor vesicles may be stored separately. Alternatively, the vesicles may be stored together so that the donor/acceptor vesicle mixture may be used directly in an assay or kit of the present invention. When donor and acceptor vesicles are stored together, the ratio of donor to acceptor vesicles is preferably in the range of from about 1:4 (donor: acceptor) to about 1:10 (donor: acceptor). Most preferably, the ratio of donor to acceptor vesicles is about 1:6. Vesicle preparations may be further stabilized by the addition of NaCl and BSA to the final concentrations of about 150 mM and 1mg/ml, respectively.

MTP may be isolated from different sources and purified using well known methods. *See e.g.,* refs. 20 and 21. For example, MTP may be isolated from bovine liver as previously described (refs. 20 and 21). Human MTP may be prepared by transfecting cultured cells with an expression vector comprising the coding sequence for MTP as described in ref. 15.

In accordance with the present invention, increases in fluorescence due to MTP-mediated lipid transfer may be measured after a short period. The methods provided herein have been successfully used to measure the MTP activity in HepG2, Caco-2 cells, and COS cells transfected with MTP expression plasmids. Furthermore, the methods provided by the present invention are useful in studying inhibition of cellular as well as purified MTP by its antagonists. The methods are amenable to automation and may be easily adopted for large-scale thorough put screening.

In addition, the present invention provides methods which may be used to assay MTP in any sample for various purposes such as identification, modulation, diagnosis etc. For example, the methods may be used to assay activity in purified MTP samples, MTP present in cell lines, tissues etc. The assays provided by the present invention are very versatile and can measure the transfer of any lipid by MTP that contains a fluorescent label. Further, the methods provided herein to measure MTP activity are simple and rapid. They are based on the determination of increases in fluorescence attributable to the binding of fluorophor with MTP that occurs during the transfer of lipids between donor and acceptor vesicles.

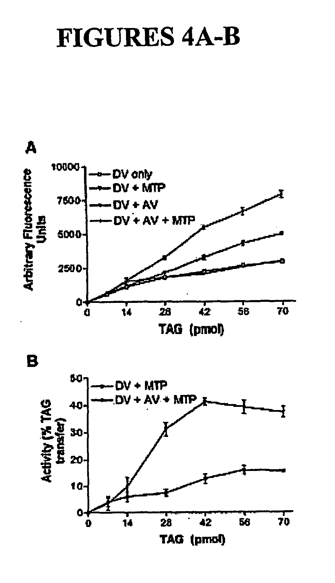

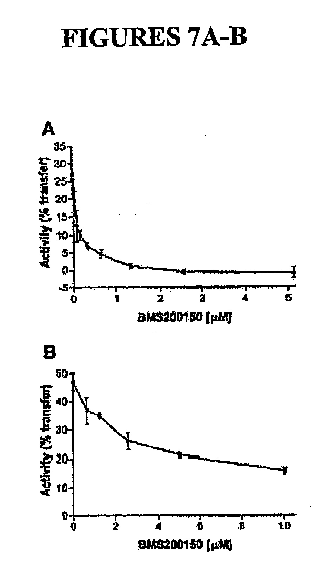

The methods of the present invention faithfully measure cellular activity in cells known to express MTP and do not measure activity in cells that do not express MTP (Table 1). Furthermore, the methods display similar inhibitory properties of antagonists that were identified using the radioisotope assay of the prior art (Fig. 7). MTP shows significantly higher activity in the presence of acceptor vesicles (Fig. 4). The low lipid binding activity of MTP in the absence of acceptor vesicles provides a unique opportunity to understand the role of different acceptor vesicles in the lipid transfer process.

Thus, the present invention provides simple and rapid fluorescence assays for the measurement of MTP activity. The advantages of the new methods include ease, rapidity, sensitivity, avoidance of the use of negatively charged lipids, versatility in studying different lipid transfer activities by purified and cellular MTP, ability to measure inhibitory activities of antagonists, and forestalling the use of radioactivity. The fluorescence assays provided by the present invention may be easily automated and used for large-scale, high-throughput screening. This approach is useful in order to identify compounds that partially inhibit MTP activity and possibly minimize the unwanted side effects related to TAG accumulation in cells. It is becoming clear that MTP is a multifunctional protein that may have functions other than being a dedicated lipoprotein assembly chaperone. Compounds identified via screening based on the fluorescence assays provided herein may be useful in the identification of other functions of MTP unrelated to lipoprotein assembly and secretion.

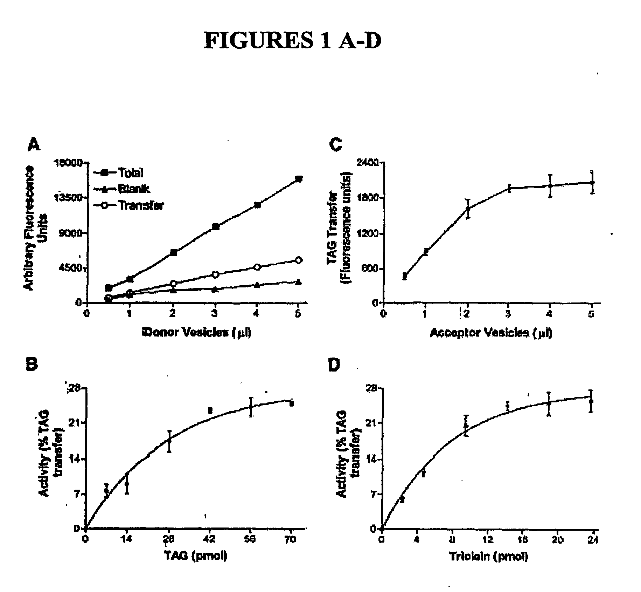

In accordance with the present disclosure, the MTP assays basically consist of three components: donor vesicles, acceptor vesicles, and MTP. The methods disclosed herein show a linear relationship with all three components of the assay mixture and time (Figs. 1-4).

A typical assay may be performed as outlined below. The amounts of the different components listed below may of course be changed, so long as the ratios among the different components remain relatively the same. Four different conditions (blank, total, positive control, and test) are recommended for each assay. In all assays, the reaction is started by the final addition of the MTP source. About 3 μl each of acceptor and donor vesicles are pipetted onto fluorescence microtiter (black) plates. About 10 μl of 10 mM Tris, pH 7.4, containing 2 mM EDTA and 10 μl of 1% BSA stock in 1.5 M NaCl are added. The exact number of vesicles for use in the assays described herein is difficult to quantify routinely. However, an easier method of quantifying vesicles is to measure phospholipid and triglycerides present in different vesicle preparations. Thus, the 3 μl of donor vesicles correlate to about 450 nmol of phosphatidylcholine (PC) and about 14 nmol of triglyceride per milliliter. A range of donor vesicle concentrations may be employed. A preferred range is e.g., anywhere from about 200 to about 600 nmol of phosphatidylcholine (PC) and 7-20 nmol triglyceride. The 3 μl of acceptor vesicles correlate to about 2,400 nmol PC/ml. However, a range of vesicle concentrations may be employed, e.g., anywhere from about 1,400 to about 3,400 nmol PC/ml.

In blanks, the needed amount of control buffer (which contains the MTP source in positive control and test samples) is added and the volume made up with water to about 100 μl. In positive controls, a known amount of the MTP source is added and the volume made up with water to about 100 μl. In tests, unknown samples are added and made up to the final assay volume. For totals, about 3 $\mu$l of donor vesicles and about 97$\mu$l of isopropanol only are added. The mixtures are incubated at about 37°C for about 30 min. Fluorescence units are measured using excitation and emission wavelengths of 460-470 and 530-550 nm, respectively. In case of low transfer activity, the incubation time can be increased. In fact, the same titer plate can be used several times to measure increases in fluorescence with time. However, the fluorophore is unstable in isopropanol over long periods of time. Thus, for periods longer than 30 min, total values from readings determine at 30 min or at earlier times should be used. The assay ingredients, including vesicles and positive controls may be made as described herein, and are also available from Chylos, Inc. (Woodbury, NY).

In a preferred embodiment of the invention, donor and acceptor vesicles may be both stored and used as combined as described above. In this embodiment, when an assay is performed, only one pipetting step of vesicles (both donor and acceptor) is needed.

The vesicles are preferably admixed with an appropriate buffer such as Tris HCl, pH at about 7.4. Other buffers may also be used such as e.g., phosphate buffer and HEPES. In a preferred embodiment, NaCl is added to a final concentration of about 150 mM. A NaCl stock solution (e.g. 3M) may be made and then diluted to yield the final concentration. Other salts such as KCl and $MgCl_2$ may also be used. BSA is added to a final concentration of about 1mg/ml in order to stabilize the vesicles. A stock solution (e.g., 20 mg BSA/ml) may be made and then diluted to yield the final concentration. In still a further embodiment, the incubation step may be performed at room temperature.

Donor vesicles containing fluorescent-lipids and acceptor vesicles may be prepared in as described in Example I. In a preferred embodiment, equal volumes of donor and acceptor vesicles may be combined. A typical assay procedure is described below:

i. Pipette in triplicate vesicles in a fluorescence microtiter (black) plate as described in the table below.
ii. Add water, sample etc. Wait for about 5 min to allow the ingredients to reach room temperature.
iii. Start the reaction by adding MTP in control and test and add isopropanol to totals.
iv. Incubate for 30 min at room temperature.

|  | Vesicles | Sample | Buffer | Water | Isopropanol | MTP |
|---|---|---|---|---|---|---|
| A. Blank | 5 $\mu$l | - | 5 $\mu$l | 90 $\mu$l | - | - |
| B. Total | 5 $\mu$l | - | - | - | 95 $\mu$l | - |
| C. Control | 5 $\mu$l | - | 5 $\mu$l | 85 $\mu$l | - | 5 $\mu$l |
| D. Test | 5 $\mu$l | 5 $\mu$l | - | 90 $\mu$l | - | - |

Measuring: the MTP activity: Measure fluorescence units (FU) using excitation and emission wavelengths of 460-470 and 530-550 nm, respectively.
Calculation of the MTP activity:

$$\text{\% Transfer in Controls (C): } (\text{Control}_{FU} - \text{Blank}_{FU}) / (\text{Total}_{FU} - \text{Blank}_{FU}) \times 100$$

$$\text{\% Transfer in Test Samples (D): } (\text{Test}_{FU} - \text{Blank}_{FU}) / (\text{Total}_{FU} - \text{Blank}_{FU}) \times 100$$

A method of identifying compounds that modulate the lipid transfer activity of MTP is also disclosed herein. The method comprises the steps of: (a) incorporating a fluorescence-labeled lipid into donor vesicles; (b) preparing acceptor vesicles; (c) mixing an aliquot of acceptor vesicles and the labeled donor vesicles with a test compound, the test compound being a known or unknown modulator of MTP; (d) adding a cellular homogenate containing MTP or isolated MTP to the mixture containing donor vesicles, acceptor vesicles, and test compound; (e) incubating a first aliquot of acceptor vesicles, labeled donor vesicles, test compound and MTP for a time and under conditions sufficient to allow binding of the fluorescence-labeled lipid with MTP during transfer of the labeled lipid from donor to acceptor vesicles; (f) incubating a second aliquot of acceptor vesicles, labeled donor vesicles and MTP for a time and under conditions sufficient to allow binding of the fluorescence-labeled lipid with MTP during transfer of the labeled lipid from donor to acceptor vesicles; (g) measuring fluorescence of the fluorescence-labeled lipid bound to MTP obtained in steps (e) and (f); and (h) correlating an increase in fluorescence of the fluorescence-labeled lipids bound to MTP obtained in step (e) when compared to the fluorescence of the fluorescence-labeled lipid bound to MTP obtained in step (f) with identification of a compound which increases lipid transfer activity of MTP, while correlating a decrease in fluorescence of the fluorescence-labeled lipid bound to MTP obtained in step (e) when compared to the fluorescence of the fluorescence-labeled lipid bound to MTP obtained in step (f) with identification of a compound which decreases lipid transfer activity of MTP.

In another aspect of the invention, there is provided a mixture for measuring the lipid transfer activity of MTP. The mixture comprises

- phosphatidylcholine (PC) acceptor vesicles,
- phosphatidylcholine (PC) donor vesicles having a fluorescence-labeled triglyceride lipid incorporated therein, and
- BSA,

wherein the concentration of phosphatidylcholine of the donor vesicles ranges from 6μM to 18μM and the concentration of fluorescence-labeled triglyceride lipid ranges from 0.21μM to 0.6μM, and
wherein the concentration of phosphatidylcholine of the acceptor vesicles ranges from 42μM to 102μM and wherein the concentration of BSA is 1mg/ml.

Preferably, the fluorescence-labeled donor vesicles are comprised of a triglyceride, a cholesterol ester, or a phospholipid. In another preferred embodiment, the triglyceride is any triacylglycerol that contains NBD label such as 1, 2, dioleoyl 3-NBD glycerol (NBD-TAG). In another preferred embodiment, the phospholipid is phosphatidylethanoloamine.

The vesicles are preferably admixed with an appropriate buffer such as Tris HCl, pH at about 7.4. Other buffers may also be used such as e.g., phosphate buffer and HEPES.

Acceptor and/or donor vesicles which form part of the mixture may be phosphatidylcholine (PC) vesicles. The vesicles are stabilized by the addition of BSA.

The following examples further describe the invention and are not meant in any way to limit the scope thereof.

### EXAMPLE I

#### MATERIALS AND METHODS

#### Materials

MTP was purified from bovine liver using the radioactivity assay (20, 21) and has been used previously (24-29). PC and TAG were from Avanti Lipids (Alabaster, AL). Fluorescence (nitrobenzoxadiazole)- labeled TAG was from Molecular Probes (Eugene, OR). Vesicles containing fluorescence-labeled cholesteryl ester (CE) and phospholipid (PL) were from Roar Biomedical, Inc. (New York, NY) and Cardiovascular Target, Inc. (New York, NY), respectively. Isopropanol and other chemicals were from Sigma Chemical Co. (St. Louis, MO). Acceptor vesicles were prepared as described by Wetterau and associates (20, 21, 30-32). Donor vesicles were also prepared according to their procedure except that cardiolipin was omitted and radiolabeled TAGs were replaced with fluorescence-labeled TAGs. Known amounts of fluorescent lipids were diluted in isopropanol to generate a standard curve, and amounts of labeled lipids in vesicles were determined after their disruption with isopropanol. The amounts of triolein in vesicles were quantified by a colorimetric assay (Infinity™ Triglyceride Reagent Kit; Sigma). The MTP inhibitor BMS200150 (diphenyl-propyl-piperidinyl-dihydroiso-indole) has been described (12) and was a kind gift from Dr. Haris Jamil of Bristol-Myers Squibb (Princeton, NJ).

#### Transfer assay

The assay was done in Microfluor ® 2 Black "U" Bottom Micro-titer ® plates (Thermo Labsystems, Franklin, MA). To the wells, was added 31 of donor (450 nmol of PC and 14 nmol of TAG per milliliter), 3 1 of acceptor (2,400 nmol PC/ml) vesicles, 101 of 10 mM Tris-HCl buffer, pH 7.4, 2 mM EDTA, 150 mM NaCl, distilled water to make the final assay volume of 1001, and purified MTP (0.1-1.5 g) in triplicate. In some experiments, NaCl and BSA were added to obtain final concentrations of 150 mM and 1 mg/ml, respectively. Plates were incubated at 37°C or at room temperature for different time periods and read with a fluorescence plate reader (7620 Microplate Fluorimeter; Cambridge Technology, Watertown, MA) using 460 nm excitation and 530 nm emission wavelengths. To determine blank values, MTP was omitted from the wells. Total fluorescence in donor vesicles was determined by adding 97 μl of isopropanol to 3 μl of donor vesicles. To study MTP inhibition, different concentrations of BMS200150 were added to the reaction mixture before the addition of MTP. MTP activity (percentage transfer) was calculated by the following equation: percentage transfer (arbitrary fluorescence units in assay wells blank values)/(total fluorescence units blank values) 100. The specific activity is expressed as percentage transfer per micrograms per hour.

Transfection of Cos-7 cells

Cos-7 cells were grown (37° C, 5% $CO_2$, humidified chamber) in DMEM (Cellgro Mediatech, Inc., Herndon, VA) supplemented with 10% fetal bovine serum and 1% antibiotic-antimycotic (Life Technologies, Rockville, MD). Cells ($1 \times 10^6$) were plated in 75 $cm^2$ flasks 24 h before transfection. At the time of transfection, cells were about 50-60% confluent. The MTP expression vector (15) pRc-hMTP (7 g; expresses human MTP under the control of the cytomegalovirus promoter) was introduced into Cos-7 cells complexed with 21 of FuGENE-6 Transfection Reagent (Roche Diagnostics, Indianapolis, IN). Cells were maintained at 37° C and 5% $CO_2$ in 7 ml of medium for about 72 h. Cos-7 cells were also treated with FuGENE-6 alone (mock transfection) and used as controls.

Determination of MTP activity in cell homogenates

MTP activity in cellular homogenates was determined as described by Jamil et al. (12). Confluent cell monolayers were washed with ice-cold sterile PBS, pH 7.4, scraped in 5 ml of PBS, transferred to 15 ml conical tubes, and pelleted down by centrifugation (2,500 rpm, 10 min, room temperature). At this point, cell pellets can be stored at 70°C. For homogenization, 750 μl of homogenization buffer (50 mM Tris-HCl, pH 7.4, 50 mM KCl, and 5 mM EDTA) and 7.5 μl of protease inhibitor cocktail (catalog number P 2714; Sigma) were added to the cell pellets. Cells were then suspended by repeated aspirations through a needle (29G, $1^{1/2}$ inches) attached to a 3 ml syringe and homogenized on ice in a ball-bearing homogenizer (clearance 0.253 inches, 10 passages). Cell homogenates were stored on ice, and protein concentrations were determined by the Bradford method (33) using Coomassie Plus Reagent (Pierce, Rockford, IL) and BSA as standards. Cell homogenates were diluted with homogenization buffer to a protein concentration of 1.5 mg/ml. MTP was released from microsomes by deoxycholate treatment (12). For this purpose, cell homogenates were adjusted to 0.054% deoxycholate by the addition of one-tenth volume of 0.54% sodium deoxycholate, pH 7.4, and left on ice for 30 min with occasional mixing. Cell membranes were subsequently removed by centrifugation in a SW55 Ti rotor at 50,000 rpm for 1 h at 10° C. The supernatants were dialyzed in 12-14 kDa cutoff dialysis bags against 15 mM Tris-HCl, pH 7.4, 40 mM NaCl, 1 mM EDTA, and 0.02% NaN3 , with the first change after 1h and the second change after 2 h followed by overnight dialysis. Cell homogenates were removed from the dialysis bag and used for protein determination and MTP assay. For inhibition studies, HepG2 cells were incubated with different concentrations of the MTP inhibitor BMS200150 for 24 h, and cell homogenates obtained from the cells were used for MTP assay.

To develop a more rapid procedure to assay cellular MTP, the procedure described by Chang, Limanek, and Chang (34) was evaluated for cell disruption. In this procedure, cells are first exposed to a hypotonic buffer and then scraped off the plates. Exposure to hypotonic buffers results in swelling of the cells, and scraping breaks these cells. Cell monolayers were washed twice with ice-cold PBS and once with 5 ml of 1 mM Tris-HCl, pH 7.6, 1 mM EGTA, and 1 mM $MgCl_2$ at 4°C. Cells were then incubated for 2 min at room temperature in 5 ml of ice-cold 1 mM Tris-HCl, pH 7.6, 1 mM EGTA, and 1 mM MgCl2. The buffer was aspirated, and 0.5 ml of the same buffer was added to cells. Cells were scraped and collected in ice-cold tubes, vortexed, and centrifuged (SW55 Ti rotor, 50,000 rpm, 10°C, 1 h), and supernatants were used for MTP assay and protein determination.

Determination of MTP activity in rat liver microsomes

Rat liver microsomes were prepared as described by Wetterau and Zilversmit (20, 21) with slight modifications. Briefly, 2 g of rat liver was cut into small pieces and washed twice with ice-cold PBS. Pieces were then homogenized in 2 ml of 50 mM Tris-HCl, pH 7.4, 5 mM EDTA, 250 mM sucrose, and 0.02% sodium azide using a Polytron homogenizer and centrifuged (Beckman micro-centrifuge, 10,900 rpm, 30 min, 4°C). Supernatants were retained and adjusted to pH 5.1 with concentrated HCl, stirred in the cold for 30 min, and centrifuged (Beckman microcentrifuge, 13,000 rpm, 30 min, 4°C). Pellets were suspended in 2 ml of 1 mM Tris-HCl, pH 7.6, 1 mM EGTA, and 1 mM $MgCl_2$ , vortexed, and ultracentrifuged (SW55 Ti rotor, 50,000 rpm, 10°C, 1 h), and supernatants were used for MTP assay and protein determination.

**EXAMPLE II**

**Optimization of assay conditions**

To standardize a fluorescence assay for MTP, TAGs were incorporated into small unilamellar PC vesicles (donor vesicles). It was anticipated that the encapsulation would result in the quenching of the fluorophore. Indeed, disruption of increasing amounts of donor vesicles with iso-propanol resulted in enhanced measurable fluorescence (Fig. 1A, total). This represents the total amounts of fluorophore present in the vesicles. Before disruption, this fluorescence is not detectable because it is quenched in vesicles. It was also envisioned that donor vesicles would be stable and would not release the fluorophore in the absence of MTP. To determine the stability, donor vesicles were mixed with acceptor vesicles and the fluorescence in the absence of MTP was measured after 30 min (Fig. 1A, blank). The blank fluorescence values ranged between 13% and 19% [15.7 $\pm$ 2.7% (average SD; n =3)] of the totals. The blank values probably represent the small leakage of the fluorophore. It was then hypothesized that the extraction of TAG from donor vesicles by MTP for transfer would manifest as increased detectable fluorescence. Incubation of constant amounts of MTP and acceptor vesicles with increasing amounts of the donor vesicles resulted in increased detection of fluorescence, indicating the transfer of TAG by MTP (Fig. 1A, transfer). The "transfer" represents the amounts of TAG being transferred by MTP between vesicles. During transfer, the MTP-bound fluorophore is most likely exposed to the aqueous environment and is now detected by the fluorimeter. The fluorescence units were 40-47% higher than the blank values. Next, the data were used to calculate the percentage transfer of TAG (Fig. 1B). The percentage transfer activity increased up to 2 $\mu$l of the donor vesicles (28 pmol of TAG) and appeared to saturate thereafter. Next, the effect of different concentrations of acceptor vesicles was studied. In these experiments, constant amounts of MTP and donor vesicles were incubated with different volumes of acceptor vesicles (Fig. 1C). The amounts of TAG transferred increased with increasing amounts of the acceptor vesicles and saturated at 2 $\mu$l. The increases in the transfer at lower concentrations of acceptor vesicles indicated that these vesicles were limiting in the assay conditions. However, at 2 $\mu$l and above, the assay became independent of the acceptor vesicle concentrations. Note that there was no decrease in detectable fluorescence with increasing concentrations of acceptor vesicles. This indicates that MTP transfers lipids between vesicles and does not cause the unidirectional deposition of lipids into the acceptor vesicles. Under these conditions, MTP was in the process of transferring 20% of the total TAG present in the donor vesicles (Fig. 1D). In subsequent studies, 3 $\mu$l of the acceptor vesicles were used.

To determine the intra-assay coefficient of variation (CV), the transfer assay was performed in 10 tubes using 0.5 g of MTP, 3 $\mu$l of donor, and 3$\mu$l of aceptor vesicles. The percentage transfer observed was 19.9 $\pm$ 1.8 (mean $\pm$ SD; n=10), and the intra-assay CV was 0.09. Similarly, the interassay variations were evaluated. Comparison of seven different independent determinations performed in triplicate using 1 $\mu$g of MTP revealed an interassay CV of 0.19. The percentage transfer observed in these experiments was 34.5 $\pm$ 3.0.

Figure 1 shows the effect of different amounts of donor and acceptor vesicles on the transfer of triacylglycerol (TAG) by microsomal triglyceride transfer protein (MTP). A: Total. Different indicated volumes of donor vesicles were disrupted by the addition of 100 $\mu$l of isopropanol, and the fluorescence units were measured immediately. Note that this value represents the total amounts of fluorophore present in the vesicles and is not a simple sum of "blank" and "transfer" values. Blank. Different volumes of donor vesicles and 3 $\mu$l of acceptor vesicles were incubated in assay buffer (100 $\mu$l) as described in Example I, Materials and Methods, and fluorescence units were measured after 30 min of incubation at 37° C. Transfer. Incubations were the same as those described for blank except that these samples also contained 0.5 g of purified MTP. This represents the amount of lipids being transported by MTP at a given time. During transfer, MTP-bound fluorescent lipids are most likely exposed to the aqueous environment and are detected by the fluorimeter. B: The data from A were used to calculate the percentage transfer of TAG as described in Materials and Methods. The TAG concentration in donor vesicles was 14 pmol/ml. C: Donor vesicles (3 $\mu$l) were incubated (30 min, 37°C) with different volumes of acceptor vesicles along with 0.5 g of purified MTP. The fluorescence units were obtained by subtracting blank fluorescence units from units observed in assay tubes. D: The data from C were used to calculate percentage transfer as described in Example I, Materials and Methods. For this purpose, blank and total fluorescence units were determined in triplicate simultaneously, as described in Example I, Materials and Methods. Line graphs and error bars represent means $\pm$ SD (n=3).

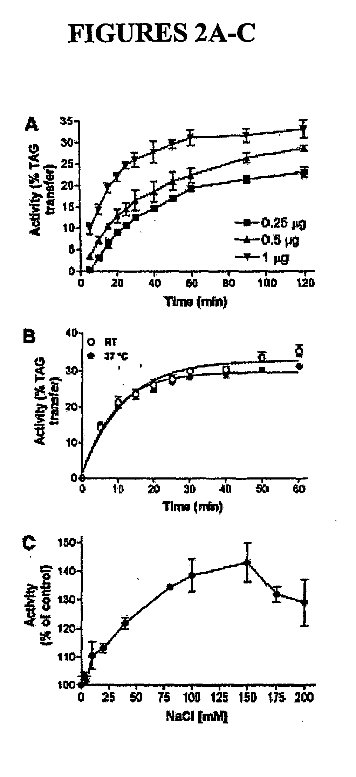

Experiments were then performed to determine the effects of time, temperature, and NaCl concentrations required for MTP activity (Fig. 2). Different indicated amounts of MTP were incubated with donor and acceptor vesicles as described in Example I, Materials and Methods. Appropriate totals and blanks were in-cluded as described in Example I, Materials and Methods. Fluorescence readings were measured at the indicated times in triplicate using a microplate reader. The percentage transfer of TAG is plotted against time. B: Temperature. The experiment was performed on two different microtiter plates. Purified MTP (0.25 g/well) was incubated with donor and acceptor vesicles in triplicate. One plate was incubated at 37° C, and the other was left at room temperature (RT). Fluorescence readings were taken at different time points at room temperature (22°C). C: Effect of NaCl. Donor vesicles (3 $\mu$l), acceptor vesicles (3 $\mu$l), and MTP (1 $\mu$g) were incubated in triplicate for 30 min in 1 mM Tris-HCl, pH 7.4, and 2 mM EDTA in the presence of various indicated concentrations of NaCl. Line graphs and error bars represent means $\pm$ SD.

At all of the different concentrations of MTP used, TAG transfer activity increased with time up to 30 min (Fig. 2A). After that time, the transfer activity began to saturate. The effect of temperature on transfer activity was studied (Fig. 2B). The lipid transfer activity of MTP was the same at room temperature (22°C) and at 37°C, indicating no significant effect of temperature on activity. These results likely indicate that MTP is optimally active at 22°C. The effect of NaCl on MTP activity (Fig. 2C) was also determined. The addition of increasing concentrations of NaCl up to 150 mM resulted in increased MTP activity. Higher concentrations of NaCl appear to inhibit transfer activity. It is concluded therefore, that 30 min incubations and 150 mM NaCl are optimum to determine MTP activity.

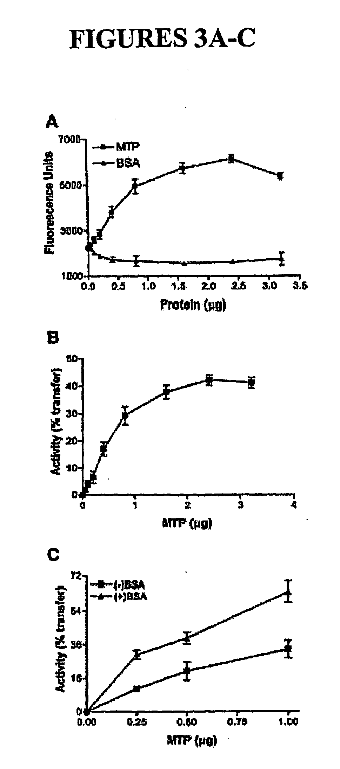

Next, the specificity of the assay using different concentrations of purified MTP and BSA on TAG transfer was studied (Fig. 3). Results shown in Figure 3A were obtained after donor [3 $\mu$l; 450 nmol of phosphatidylcholine (PC) and 14 nmol of TAG per milliliter] and acceptor (3 $\mu$l; 2,400 nmol PC/ml) vesicles were incubated with different indicated amounts of MTP or BSA in triplicate for 30 min. Fluorescence units were obtained after subtracting blanks from the assay tubes. Data from these results (Figure 3A) for MTP were used to determine percentage transfer activity. For BSA, these values were negative and were not plotted. The assay was also performed in the absence and presence of BSA (0.1%) using various indicated amounts of purified MTP (Figure 3C) Line graphs and error bars represent means $\pm$ SD.

The addition of increasing amounts of MTP resulted in enhanced detectable fluorescence (Fig. 3A). In contrast, the presence of BSA decreased the amounts of detectable fluorescence and may represent either quenching of the released fluorescence by BSA or stabilization of the donor vesicles by BSA, preventing the basal fluorophore leakage. In Fig. 3B, the data were converted to measure the percentage of TAG undergoing transfer between vesicles. Under the experimental conditions, the amounts of TAG being transferred reached saturation at 2 $\mu$g of MTP. At saturation, 40% of the total TAG was in the process of transfer and probably represented the maximum binding capacity of MTP.

Because BSA decreased the fluorescence units in blank samples, it was reasoned that it may have a positive effect on MTP assay. Furthermore, the presence of BSA may decrease the loss of MTP and vesicles by adsorption to tube surfaces. To test this hypothesis, BSA (1 mg/ml) was used in the assay. As shown in Fig. 3C, the activity measured in the presence of BSA was almost twice that observed in the absence of BSA. This was mainly attributable to decreased blank values in the presence of BSA. The blank values in the presence and absence of BSA were 12.5 $\pm$ 0.7% and 18.5 $\pm$ 0.5% (n=3) of the totals. Thus, the inclusion of BSA improves the sensitivity of the assay, most likely by preventing the leakage of the fluorophore from the donor vesicles.

Subsequently, the specific activity of MTP determined by radiolabel and fluorescence assays was compared. The specific activity (percentage transfer per microgram per hour) in various preparations using the radiolabel assay was 12.5 $\pm$ 2.4 (n=27). The specific activity by the fluorescence method in the absence of BSA was 92.6 $\pm$ 19.7 (n=20), whereas the specific activity determined in the presence of BSA was 204.4 $\pm$ 33.1 (n=7). Thus, specific activities measured by the fluorescence assay were higher than those observed using the radiolabel assay. The higher specific activities may be attributable to the higher sensitivity of the assay. Another reason for the difference may be different parameters used in these two assays. In the radiolabeled assay, the amount of TAG transferred to acceptor vesicles is measured. In contrast, the fluorescence assay described in this example measures the amount of TAG being transferred by the MTP.

**TABLE 1**

Determination of MTP activity in cells using the deoxycholate method

| Cells | Protein per Assay | Fluorescence | Specific Activity |
|---|---|---|---|
| | $\mu$g | % change | % transfer/ $\mu$g/h |
| Cos-7 cells | 70.9 | 0.9 $\pm$ 0.6 | 0.026 $\pm$ 0.017 |
| Cos-7 cells + MTP[a] | 51.0 | 10.2 $\pm$ 1.0 | 0.401 $\pm$ 0.04 |
| HepG2 cells | 47.2 | 23.2 $\pm$ 0.6 | 0.982 $\pm$0.024 |
| Caco-2 cells | 48.6 | 19.5 $\pm$ 1.4 | 0.801 $\pm$ 0.057 |

MTP, microsomal triglyceride transfer protein.
[a] Cos-7 cells transiently transfected with MTP expression vectors.

**Role of acceptor vesicles**

The MTP assay consists of three components: donor vesicles, acceptor vesicles, and MTP. Obviously, donor vesicles and MTP are required. It was reasoned that MTP could transfer lipids between donor vesicles and that the acceptor vesicles may not be needed for activity measurements. To test this hypothesis, different amounts of donor vesicles (450 nmol of PC and 14 nmol of TAG per milliliter) were incubated without acceptor vesicles and MTP (DV only), with 0.5 g of purified MTP (DV MTP), with 3 $\mu$l of acceptor vesicles (DV + AV), or with 3 $\mu$l of acceptor vesicles (2,400 nmol PC/ml) and 0.5 $\mu$g of purified MTP (DV + AV + MTP) in assay buffer (100 $\mu$l). Fluorescence units were measured after 30 min of incubation at 37°C. This data is plotted in Figure 4A.

The data shown in Figure 4A were used to calculate percentage transfer as described in Materials and Methods. Line graphs and error bars represent means $\pm$ SD.

Incubation of increasing amounts of donor vesicles with (DV + AV) or without (DV only) acceptor vesicles gave similar fluorescent readings, indicating little transfer of TAG in the absence of MTP (Fig. 4A). These data are in agreement with the blanks in Fig. 1A. Incubation of increasing amounts of donor vesicles with MTP (DV + MTP) resulted in some increase in fluorescence, indicating some transfer of lipids. In contrast, a significant increase in fluorescence was observed when acceptor vesicles were included in the reaction mixture (DV + AV + MTP). The data were then used to calculate percentage transfer of TAG (Fig. 4B). In the absence of acceptor vesicles (DV + MTP), the TAG transfer ranged between 4% and 16%. In the presence of acceptor vesicles (DV + AV + MTP), however, MTP was engaged in transferring almost 40% of TAG present in donor vesicles. These data demonstrate that the presence of acceptor vesicles greatly facilitates the transfer process.

**Transfer of different lipids to various acceptors by MTP**

The effect of different types of acceptor vesicles on the transfer of various lipids by MTP was studied. First, PC or PC/TAG vesicles were used as acceptors. The percentage transfers observed in triplicate with these acceptors were $33 \pm 2.6$ and $30.9 \pm 1.3$, respectively, indicating that the absence of TAG in the acceptor vesicles does not have any significant effect on the transfer activity.

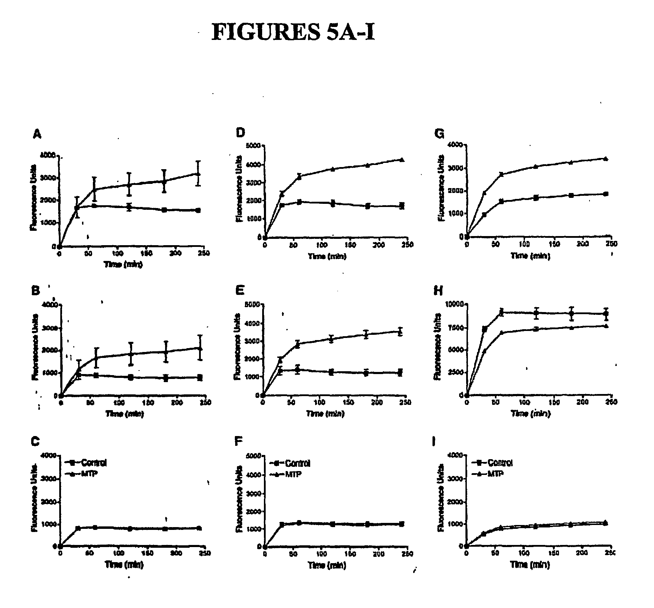

MTP is known to transfer other lipids besides TAG (20). Thus, experiments were performed to evaluate the suitability of this assay to study the transfer of CEs and PLs in addition to TAG (Fig. 5). In this experiment, small unilamellar vesicles (PC/TAG vesicles), apoB lipoproteins (which contained both VLDL and LDL), and HDL were used as acceptors and the transfer was studied over a period of 4 h. This experiment was performed before the optimization of conditions described in Fig. 2. In these early experiments, incubations were performed for a longer period of time before measuring the transfer activity.

Three different types of donor vesicles (3 $\mu$l) containing TAG (A-C), cholesteryl ester (D-F), or phospholipids (G-I) were used. The acceptor vesicles (3 $\mu$l; 2,400 nmol PC/ml) were small unilamellar vesicles (PC/TAG vesicles; A, D, and G), apolipoprotein B (apoB) lipoproteins (VLDL and LDL; B, E, and H), and HDLs (10 mg protein/ml; C, F, and I). Different donor and acceptor vesicles were incubated without (control) or with 1 $\mu$g of purified MTP (MTP) in triplicate for the indicated times, and fluorescence was measured as described in Materials and Methods. Line graphs and error bars represent means $\pm$ SD.

Results indicated that MTP transferred TAG when donor vesicles were incubated with small unilamellar PC/TAG vesicles (Fig. 5A) and apoB lipoproteins (Fig. 5B) but not when incubated with HDL (Fig. 5C). The transfer resulted in a 109-172% increase in fluorescence units after 4 h of incubation (Fig. 5A, B). Similarly, MTP transferred CE in the presence of PC/TAG vesicles (Fig. 5D) and apoB lipoproteins (Fig. 5E) but not in the presence of HDL (Fig. 5F). MTP was able to transfer PLs when donor vesicles were incubated with PC/TAG vesicles; the increase in fluorescence was 186% at 4 h (Fig. 5G). However, studies of the transfer of PL by MTP in the presence of apoB lipoproteins as acceptors were difficult to interpret (Fig. 5H). This was attributable to a significant increase in the background fluorescence when donor vesicles were incubated with apoB lipoproteins in the absence of MTP (compare controls in Fig. 5G, H; also note the different $y$ values in these panels). Again, MTP did not transfer PL when HDL was used as an acceptor (Fig. 5I). These studies show that MTP activity transfers both neutral lipids, TAG and CE, when small unilamellar vesicles and apoB lipoproteins are used as acceptors.

**Measurement of MTP activity in cells**

The next question was to determine whether this assay could be used to measure MTP activity in cells **(Table 1).** Microsomal MTP was released by deoxycholate treatment described by Jamil et al. (12). Cos-7 cells were transfected or not with MTP expression vector to determine the specificity of the MTP assay (Table 1). As expected, no MTP was detectable in mock-transfected Cos-7 cells. However, transfection with MTP expression vectors resulted in increased MTP activity in Cos-7 cells, in agreement with other studies (15, 29). Similarly, MTP activity could be measured in cellular homogenates of HepG2 and differentiated Caco-2 cells. These studies indicate that the new method can be used to determine cellular MTP activity. The deoxycholate method is cumbersome, involves several steps, and requires at least 2 days. To simplify this procedure, the method of Chang, Limanek, and Chang (34) was evaluated, which involves the disruption of cells by hypotonic buffers and requires far less time to prepare cell extracts for assay.

Rat liver microsomes were subjected to hypotonic buffer treatment, and released contents were used for MTP activity measurements. The specific activity (percentage transfer per microgram per hour) of MTP in liver microsomal contents was $0.498 \pm 0.09$ (mean $\pm$ SD, n=9).

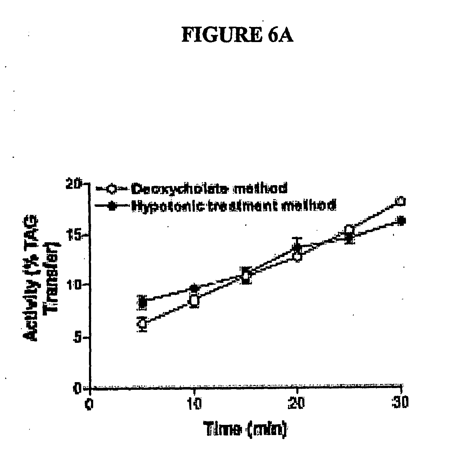

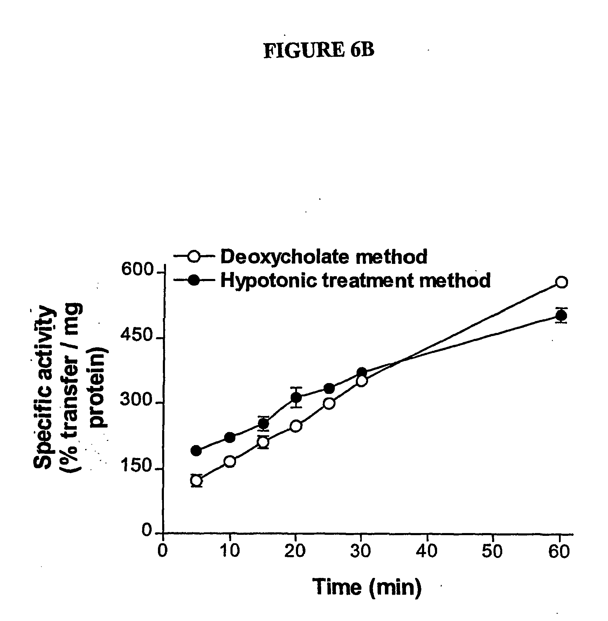

For transfer assays, 40-50 g of cellular proteins were used in triplicate, and the activity was measured for up to 1 hr. As shown in Figures 6 and 10, both methods gave similar MTP activity. Thus, hypotonic treatment is a better procedure to measure cellular MTP activity because less time and fewer manipulations are required.

**Inhibition of MTP activity**

Experiments were then performed to measure the effect of MTP antagonists on its activity (Fig. 7). For this purpose, an MTP inhibitor, BMS200150, described by Jamil et al. (12) was used. Purified MTP (1 g) was incubated with donor and acceptor vesicles for 30 min in the presence of the indicated concentrations of the MTP inhibitor BMS200150. As shown in Fig. 7A, increasing concentrations of BMS200150 resulted in a dose-dependent inhibition of the purified MTP activity. The $IC_{50}$ was 0.08 M and was in the range reported by others (12).

Next, HepG2 cells were incubated with different concentrations of the inhibitor for 24 h, and homogenates were prepared using the deoxycholate method (12) and assayed for MTP activity. Cells were washed, and lysates were prepared using the deoxycholate method described in Materials and Methods. For transfer assays, 40-50 g of cellular proteins was used in triplicate. As shown in Fig. 7B, increasing concentrations of BMS200150 resulted in decreased cellular MTP activity. The $IC_{50}$ value of ~1.3 M is in agreement with published studies (12). The differences in the $IC_{50}$ values obtained for the purified MTP and cell lysates may be attributable to the presence of other proteins in cell lysates that might interact with the inhibitor and decrease its efficacy. These studies indicate that the assay is useful in measuring MTP activity and its inhibition by antagonists.

REFERENCES

1. Hussain, M. M., J. Shi, and P. Dreizen. 2003. Microsomal triglyceride transfer protein and its role in apolipoprotein B-lipoprotein assembly. J. Lipid Res. 44: 22-32.

2. Hussain, M. M., J. Iqbal, K. Anwar, P. Rava, and K. Dai. 2003. Microsomal triglyceride transfer protein: a multifunctional protein. Front. Biosci. 8: S500-S506.

3. Bakillah, A., and A. El Abbouyi. 2003. The role of microsomal triglyceride transfer protein in lipoprotein assembly: an update. Front. Biosci. 8: D294-D305.

4. Shelness, G. S., and J. A. Sellers. 2001. Very-low-density lipoprotein assembly and secretion. Curr. Opin. Lipidol. 12: 151-157.

5. Gordon, D. A., and H. Jamil. 2000. Progress towards understanding the role of microsomal triglyceride transfer protein in apolipo-protein-B lipoprotein assembly. Biochim. Biophys. Acta. 1486: 72-83.

6. Wetterau, J. R., M. C. M. Lin, and H. Jamil. 1997. Microsomal triglyceride transfer protein. Biochim. Biophys. Acta. 1345: 136-150.

7. Hussain, M. M. 2000. A proposed model for the assembly of chylomicrons. Atherosclerosis. 148: 1-15.

8. Davis, R. A. 1999. Cell and molecular biology of the assembly and secretion of apolipoprotein B-containing lipoproteins by the liver. Biochim. Biophys. Acta. 1440: 1-31.

9. Fisher, E. A., and H. N. Ginsberg. 2002. Complexity in the secretory pathway: the assembly and secretion of apolipoprotein B-containing lipoproteins. J. Biol. Chem. 277: 17377-17380.

10. Wetterau, J. R., L. P. Aggerbeck, M-E. Bouma, C. Eisenberg, A. Munck, M. Hermier, J. Schmitz, G. Gay, D. J. Rader, and R. E. Gregg. 1992. Absence of microsomal triglyceride transfer protein in individuals with abetalipoproteinemia. Science. 258: 999-1001.

11. Shoulders, C. C., D. J. Brett, J. D. Bayliss, T. M. E. Narcisi, A. Jarmuz, T. T. Grantham, P. R. D. Leoni, S. Bhattacharya, R. J. Pease, P. M. Cullen, S. Levi, P. G. H. Byfield, P. Purkiss, and J. Scott. 1993. Abetalipoproteinemia is caused by defects of the gene encoding the 97 kDa subunit of a microsomal triglyceride transfer protein. Hum. Mol. Genet. 2: 2109-2116.

12. Jamil, H., D. A. Gordon, D. C. Eustice, C. M. Brooks, J. K. Dickson, Jr., Y. Chen, B. Ricci, C-H. Chu, T. W. Harrity, C. P. Ciosek, Jr., S. A. Biller, R. E. Gregg, J. R. Wetterau. 1996. An inhibitor of the microsomal triglyceride transfer protein inhibits apoB secretion from HepG2 cells. Proc. Natl. Acad. Sci. USA. 93: 11991-11995.

13. Haghpassand, M., D. Wilder, and J. B. Moberly. 1996. Inhibition of apolipoprotein B and triglyceride secretion in human hepatoma cells (HepG2). J. Lipid Res. 37: 1468-1480.

14. Benoist, F., and T. Grand-Perret. 1997. Co-translational degradation of apolipoprotein B100 by the proteasome is prevented by microsomal triglyceride transfer protein-synchronized translation studies on HepG2 cells treated with an inhibitor of microsomal triglyceride transfer protein. J. Biol. Chem. 272: 20435-20442.

15. Gordon, D. A., H. Jamil, D. Sharp, D. Mullaney, Z. Yao, R. E. Gregg, and J. Wetterau. 1994. Secretion of apolipoprotein B-containing lipoproteins from HeLa cells is dependent on expression of the microsomal triglyceride transfer protein and is regulated by lipid availability. Proc. Natl. Acad. Sci. USA. 91: 7628-7632.

16. Leiper, J. M., J. D. Bayliss, R. J. Pease, D. J. Brett, J. Scott, and C. C. Shoulders. 1994. Microsomal triglyceride transfer protein, the abetalipoproteinemia gene product, mediates the secretion of apolipoprotein B-containing lipoproteins from heterologous cells. J. Biol. Chem. 269: 21951-21954.

17. Raabe, M., M. M. Véniant, M. A. Sullivan, C. H. Zlot, J. Björkegren, L. B. Nielsen, J. S. Wong, R. L. Hamilton, and S. G. Young. 1999. Analysis of the role of microsomal triglyceride transfer protein in the liver of tissue-specific knockout mice. J. Clin. Invest. 103: 1287-1298.

18. Wang, Y. W., K. Tran, and Z. M. Yao. 1999. The activity of microsomal triglyceride transfer protein is essential for accumulation of triglyceride within microsomes in McA-RH7777 cells-a unified model for the assembly of very low density lipoproteins. J. Biol. Chem. 274: 27793-27800.

19. Kulinski, A., S. Rustaeus, and J. E. Vance. 2002. Microsomal triacylglycerol transfer protein is required for lumenal accretion of triacylglycerol not associated with Apo B, as well as for Apo B lipidation. J. Biol. Chem. 277: 31516-31525.

20. Wetterau, J. R., and D. B. Zilversmit. 1984. A triglyceride and cholesteryl ester transfer protein associated with liver microsomes. J. Biol. Chem. 259: 10863-10866.

21. Wetterau, J. R., and D. B. Zilversmit. 1985. Purification and characterization of microsomal triglyceride and cholesteryl ester transfer protein from bovine liver microsomes. Chem. Phys. Lipids. 38: 205-222.

22. Jamil, H., J. K. Dickson, Jr., C-H. Chu, M. W. Lago, J. K. Rinehart, S. A. Biller, R. E. Gregg, and J. R. Wetterau. 1995. Microsomal triglyceride transfer protein. Specificity of lipid binding and transport. J. Biol. Chem. 270: 6549-6554.

23. Chang, G., R. B. Ruggeri, and H. J. Harwood, Jr. 2002. Microsomal triglyceride transfer protein (MTP) inhibitors: discovery of clinically active inhibitors using high-throughput screening and parallel synthesis paradigms. Curr. Opin. Drug Discov. Dev. 5: 562-570.

24. Hussain, M. M., A. Bakillah, and H. Jamil. 1997. Apolipoprotein B binding to microsomal triglyceride transfer protein decreases with increases in length and lipidation: implications in lipoprotein bio-synthesis. Biochemistry. 36: 13060-13067.

25. Hussain, M. M., A. Bakillah, N. Nayak, and G. S. Shelness. 1998. Amino acids 430-570 in apolipoprotein B are critical for its binding to microsomal triglyceride transfer protein. J. Biol. Chem. 273:25612-25615.

26. Bakillah, A., H. Jamil, and M. M. Hussain. 1998. Lysine and arginine residues in the N-terminal 18% of apolipo-protein B are critical for its binding to microsomal triglyceride transfer protein. Biochemistry. 37: 3727-3734.

27. Bakillah, A., N. Nayak, U. Saxena, R. M. Medford, and M. M. Hussain. 2000. Decreased secretion of apoB follows inhibition of apoB-MTP binding by a novel antagonist. Biochemistry. 39: 4892-4899.

28. Bakillah, A., and M. M. Hussain. 2001. Binding of microsomal triglyceride transfer protein to lipids results in increased affinity for apolipoprotein B: Evidence for stable microsomal MTP-lipid complexes. J. Biol. Chem. 276: 31466-31473.

29. Tietge, U. J. F., A. Bakillah, C. Maugeais, K. Tsukamoto, M. M. Hussain, and D. J. Rader. 1999. Hepatic overexpression of microsomal triglyceride transfer protein (MTP) results in increased in vivo secretion of VLDL triglycerides and apolipoprotein B. J. Lipid Res.40: 2134-2139.

30. Wetterau, J. R., L. P. Aggerbeck, P. M. Laplaud, and L. R. McLean. 1991. Structural properties of the microsomal triglyceride-transfer protein complex. Biochemistry. 30: 4406-4412.

31. Atzel, A., and J. R. Wetterau. 1993. Mechanism of microsomal triglyceride transfer protein catalyzed lipid transport. Biochemistry. 32: 10444-10450.

32. Atzel, A., and J. R. Wetterau. 1994. Identification of two classes of lipid molecule binding sites on the microsomal triglyceride transfer protein. Biochemistry. 33: 15382-15388.

33. Bradford, M. M. 1976. A rapid and sensitive method for the quantitation of microgram quantities of protein utilizing the principle of protein-dye binding. Anal. Biochem. 72: 248-254.

34. Chang, T. Y., J. S. Limanek, and C. C. Chang. 1981. A simple and efficient procedure for the rapid homogenization of cultured animal cells grown in monolayer. Anal. Biochem. 116: 298-302.

**Claims**

1. A method of measuring levels of microsomal triglyceride transfer protein (MTP), the method comprising:

    (a) preparing phosphatidylcholine (PC)donor vesicles having a fluorescence-labeled triglyceride lipid incorporated therein;
    (b) preparing phosphatidylcholine (PC) acceptor vesicles,
    (c) incubating either a cellular homogenate containing MTP or isolated MTP with the acceptor vesicles and the labeled donor vesicles for a time and under conditions sufficient to allow binding of the fluorescence-labeled triglyceride lipid with MTP during transfer of the labeled lipid from donor to acceptor vesicles; wherein the final concentration of phosphatidylcholine of the donor vesicles ranges from $6\mu M$ to $18\mu M$ and the final concentration of fluorescence-labeled triglyceride lipid ranges from $0.21\mu M$ to $0.6\mu M$, and wherein the final concentration of phospholipid phosphatidylcholine of the acceptor vesicles ranges from 42μM to 102μM, (d) measuring fluorescence of the fluorescence-labeled triglyceride lipid bound to the MTP, wherein the vesicles are stabilized by the addition of BSA to the reaction mixture in a concentration of 1mg/ml.

2. The method of claim 1, wherein the final concentration of phosphatidylcholine of the donor vesicles is 13.5μM and the final concentration of fluorescence-labeled triglyceride lipid is 0.42μM, and/or, wherein the final concentration of phospholipid phosphatidylcholine of the acceptor vesicles is 72μM.

3. The method of claim 1 or 2, wherein the cellular homogenate comprises animal cells that express - MTP.

4. The method of claim 1 or 2, wherein the cellular homogenate comprises cells from insects or microorganisms that express MTP.

5. The method of claim 4 wherein the fluorescence-labeled triglyceride is triacylglycerol (TAG).

6. The method of claim 4 or 5, wherein the fluorescence-labeled triglyceride is any triacylglycerol that contains at least one NBD.

7. The method of claim 5, wherein the fluorescence-labeled TAG contains at least one NBD at any position and fatty acids are located at other positions on the TAG.

8. The method of any of claims 5-7, wherein the fluorescence-labeled triacylglycerol is 1, 2 dioleoyl 3-NBD glycerol.

9. A method of identifying compounds that modulate the lipid transfer activity of MTP, said method comprising:

(a) incorporating a fluorescence-labeled triglyceride lipid into phosphatidylcholine (PC) donor vesicles;
(b) preparing phosphatidylcholine (PC) acceptor vesicles;
(c) mixing an aliquot of acceptor vesicles and the labeled donor vesicles with a test compound, the test compound being a known or unknown modulator of MTP;
(d) adding a cellular homogenate containing MTP or isolated MTP to the mixture containing donor vesicles, acceptor vesicles, and test compound;
(e) incubating a first aliquot of acceptor vesicles, labeled donor vesicles, test compound and MTP for a time and under conditions sufficient to allow binding of the fluorescence-labeled triglyceride lipid with MTP during transfer of the labeled lipid from donor to acceptor vesicles; wherein the concentration of phosphatidylcholine of the donor vesicles ranges from 6μM to 18 μM and the concentration of fluorescence-labeled triglyceride lipid ranges from 0.21μM to 0.6μM, and wherein the concentration of phospholipid phosphatidylcholine of the acceptor vesicles ranges from 42μM to 102μM, wherein the vesicles are stabilized by the addition of 1mg/ml BSA to the reaction mixture;
(f) incubating a second aliquot of acceptor vesicles, labeled donor vesicles and MTP for a time and under conditions sufficient to allow binding of the fluorescence-labeled triglyceride lipid with MTP during transfer of the labeled lipid from donor to acceptor vesicles; wherein the concentration of phospholipid phosphatidylcholine of the donor vesicles ranges from 6μM to 18μM and the concentration of fluorescence-labeled triglyceride lipid ranges from 0.21μM to 0.6μM , and wherein the concentration of phospholipid phosphatidylcholine of the acceptor vesicles ranges from 42μM to 102μM, wherein the vesicles are stabilized by the addition of 1mg/ml BSA to the reaction mixture;
(g) measuring fluorescence of the fluorescence-labeled triglyceride lipid bound to MTP obtained in steps (e) and (f); and
(h) correlating an increase in fluorescence of the fluorescence-labeled triglyceride lipids bound to MTP obtained in step (e) when compared to the fluorescence of the fluorescence-labeled triglyceride lipid bound to MTP obtained in step (f) with identification of a compound which increases lipid transfer activity of MTP, while correlating a decrease in fluorescence of the fluorescence-labeled triglyceride lipid bound to MTP obtained in step (e) when compared to the fluorescence of the fluorescence-labeled triglyceride lipid bound to MTP obtained in step (f) with identification of a compound which decreases lipid transfer activity of MTP.

10. The method of claim 9, wherein the concentration of phosphatidylcholine of the donor vesicles is 13.5μM and the concentration of the fluorescence-labeled triglyceride lipid is 0.42μM, and/or, wherein the concentration of phosphatidylcholine of the acceptor vesicles is 72μM.

11. The method of claim 9 or 10, wherein the cellular homogenate comprises animal cells that express MTP.

12. The method of any one of claims 9 to 11, wherein the cellular homogenate comprises cells from insects or microorganisms that express MTP.

13. The method of any of claims 9 to 12 wherein the fluorescence-labeled triglyceride is triacylglycerol (TAG).

14. The method of claim 12 or 13, wherein the fluorescence-labeled triglyceride is any triacylglycerol that contains at least one NBD.

15. The method of claim 14, wherein the fluorescence-labeled TAG contains at least one NBD at any position and fatty acids are located at other positions on the TAG.

16. The method of any of claims 14 to 15, wherein the fluorescence-labeled triacylglycerol is. 1, 2 dioleoyl 3-NBD glycerol.

17. A mixture for measuring the lipid transfer activity of MTP according to the method of any one of the previous claims, said mixture comprising:

   - phosphatidylcholine (PC) acceptor vesicles,
   - phosphatidylcholine (PC) donor vesicles having a fluorescence-labeled triglyceride lipid incorporated therein, and
   - BSA,

   wherein the concentration of phosphatidylcholine of the donor vesicles ranges from $6\mu M$ to $18\mu M$ and the concentration of fluorescence-labeled triglyceride lipid ranges from $0.21\mu M$ to $0.6\mu M$, and wherein the concentration of phosphatidylcholine of the acceptor vesicles ranges from $42\mu M$ to $102\mu M$ and wherein the concentration of BSA is 1mg/ml.

18. The mixture of claim 17, wherein the concentration of phosphatidylcholine of the donor vesicles is $13.5\mu M$ and the concentration of the fluorescence-labeled TAG is $0.42\mu M$, and/or, wherein the concentration of phosphatidylcholine of the acceptor vesicles is $72\mu M$.

19. The mixture of claim 18, wherein the fluorescence-labeled triglyceride is triacylglycerol (TAG).

20. The mixture of claims 18 or 19, wherein the fluorescence-labeled triglyceride is any triacylglycerol that contains at least one NBD.

21. The mixture of claim 20, wherein the fluorescence-labeled TAG contains at least one NBD at any position and fatty acids are located at other positions on the TAG.

22. The mixture of any of claims 17 to 21, wherein the fluorescence-labeled triacylglycerol is 1, 2, dioleoyl 3-NBD glycerol.


**Patentansprüche**

1. Verfahren zum Messen von Werten von mikrosomalem Triglyceridtransferprotein (MTP), wobei das Verfahren umfasst:

   (a) Herstellen von Phosphatidylcholin- (PC-) Donorvesikeln mit einem darin eingearbeiteten Fluoreszenz-markierten Triglyceridlipid;
   (b) Herstellen von Phosphatidylcholin- (PC-) Akzeptorvesikeln,
   (c) Inkubieren entweder eines zellulären Homogenats, das MTP enthält, oder eines isolierten MTP mit den Akzeptorvesikeln und den markierten Donorvesikeln für eine Zeit und unter Bedingungen, die ausreichen, um eine Bindung des Fluoreszenz-markierten Triglyceridlipids mit MTP während des Transfers des markierten Lipids von Donor- zu Akzeptorvesikeln zu ermöglichen; wobei die Endkonzentration von Phosphatidylcholin der Donorvesikel von $6\ \mu M$ bis $18\ \mu M$ reicht und die Endkonzentration von Fluoreszenz-markiertem Triglyceridlipid von $0,21\ \mu M$ bis $0,6\ \mu M$ reicht, und wobei die Endkonzentration von Phospholipid-Phosphatidylcholin der Akzeptorvesikel von $42\ \mu M$ bis $102\ \mu M$ reicht,

(d) Messen der Fluoreszenz des Fluoreszenz-markierten Triglyceridlipids, das an das MTP gebunden ist,

wobei die Vesikel durch die Zugabe zu dem Reaktionsgemisch von BSA in einer Konzentration von 1 mg/ml stabilisiert sind.

2. Verfahren nach Anspruch 1, wobei die Endkonzentration von Phosphatidylcholin der Donorvesikel 13,5 μM beträgt und die Endkonzentration von Fluoreszenz-markiertem Triglyceridlipid 0,42 μM beträgt und/oder wobei die Endkonzentration von Phospholipid-Phosphatidylcholin der Akzeptorvesikel 72 μM beträgt.

3. Verfahren nach Anspruch 1 oder 2, wobei das zelluläre Homogenat Tierzellen umfasst, die MTP exprimieren.

4. Verfahren nach Anspruch 1 oder 2, wobei das zelluläre Homogenat Zellen von Insekten oder Mikroorganismen umfasst, die MTP exprimieren.

5. Verfahren nach Anspruch 4, wobei das Fluoreszenz-markierte Triglycerid Triacylglycerol (TAG) ist.

6. Verfahren nach Anspruch 4 oder 5, wobei das Fluoreszenz-markierte Triglycerid jedes Triacylglycerol ist, das mindestens eine NBD enthält.

7. Verfahren nach Anspruch 5, wobei das Fluoreszenz-markierte TAG mindestens eine NBD an einer beliebigen Position enthält und Fettsäuren sich an anderen Positionen auf dem TAG befinden.

8. Verfahren nach einem der Ansprüche 5 bis 7, wobei das Fluoreszenz-markierte Triacylglycerol 1,2-Dioleoyl-3-NBD-glycerol ist.

9. Verfahren zur Identifizierung von Verbindungen, die die Lipidtransferaktivität von MTP modulieren, wobei das Verfahren umfasst:

(a) Einarbeiten eines Fluoreszenz-markierten Triglyceridlipids in Phosphatidylcholin- (PC-) Donorvesikel;
(b) Herstellen von Phosphatidylcholin- (PC-) Akzeptorvesikel;
(c) Mischen eines Aliquots von Akzeptorvesikeln und der markierten Donorvesikel mit einer Testverbindung, wobei die Testverbindung ein bekannter oder unbekannter Modulator von MTP ist;
(d) Zugeben eines zellulären Homogenats, das MTP enthält, oder eines isolierten MTP zu dem Gemisch, das Donorvesikel, Akzeptorvesikel und Testverbindung enthält;
(e) Inkubieren eines ersten Aliquots von Akzeptorvesikeln, markierten Donorvesikeln, Testverbindung und MTP für eine Zeit und unter Bedingungen, die ausreichen, um eine Bindung des Fluoreszenz-markierten Triglyceridlipids mit MTP während des Transfers des markierten Lipids von Donor- zu Akzeptorvesikeln zu ermöglichen; wobei die Konzentration von Phosphatidylcholin der DonorVesikel von 6 μM bis 18 μm reicht und die Konzentration von Fluoreszenz-markiertem Triglyceridlipid von 0,21 μM bis 0,6 μM reicht, und wobei die Konzentration von Phospholipid-Phosphatidylcholin der Akzeptorvesikel von 42 μM bis 102 μM reicht, wobei die Vesikel durch die Zugabe von 1 mg/ml BSA zu dem Reaktionsgemisch stabilisiert sind;
(f) Inkubieren eines zweiten Aliquots von Akzeptorvesikeln, markierten Donorvesikeln und MTP für eine Zeit und unter Bedingungen, die ausreichen, um eine Bindung des Fluoreszenz-markierten Triglyceridlipids mit MTP während des Transfers des markierten Lipids von Donor- zu Akzeptorvesikeln zu ermöglichen; wobei die Konzentration von Phospholipid-Phosphatidylcholin der Donorvesikel von 6 μM bis 18 μM reicht und die Konzentration von Fluoreszenz-markiertem Triglyceridlipid von 0,21 μM bis 0,6 μM reicht, und wobei die Konzentration von Phospholipid-Phosphatidylcholin der Akzeptorvesikel von 42 μM bis 102 μM reicht, wobei die Vesikel durch Zugabe von 1 mg/ml BSA zu dem Reaktionsgemisch stabilisiert sind;
(g) Messen der Fluoreszenz des Fluoreszenz-markierten Triglyceridlipids, das an MTP gebunden ist, das in den Schritten (e) und (f) erhalten wurde; und
(h) Korrelieren eines Anstiegs in der Fluoreszenz der Fluoreszenz-markierten Triglyceridlipide, die an MTP gebunden sind, das in Schritt (e) erhalten wurde, im Vergleich zu der Fluoreszenz des Fluoreszenz-markierten Triglyceridlipids, das an MTP gebunden ist, das in Schritt (f) erhalten wurde, mit einer Identifizierung einer Verbindung, die die Lipidtransferaktivität von MTP erhöht, und Korrelieren einer Abnahme in der Fluoreszenz des Fluoreszenz-markierten Triglyceridlipids, das an MTP gebunden ist, das in Schritt (e) erhalten wurde, im Vergleich zu der Fluoreszenz des Fluoreszenz-markierten Triglyceridlipids, das an MTP gebunden ist, das in Schritt (f) erhalten wurde, mit einer Identifizierung einer Verbindung, die die Lipidtransferaktivität von MTP senkt.

**10.** Verfahren nach Anspruch 9, wobei die Konzentration von Phosphatidylcholin der Donorvesikel 13,5 $\mu$M beträgt und die Konzentration von Fluoreszenz-markiertem Triglyceridlipid 0,42 $\mu$M beträgt und/oder wobei die Konzentration von Phosphatidylcholin der Akzeptorvesikel 72 $\mu$M beträgt.

**11.** Verfahren nach Anspruch 9 oder 10, wobei das zelluläre Homogenat Tierzellen umfasst, die MTP exprimieren.

**12.** Verfahren nach einem der Ansprüche 9 bis 11, wobei das zelluläre Homogenat Zellen von Insekten oder Mikroorganismen umfasst, die MTP exprimieren.

**13.** Verfahren nach einem der Ansprüche 9 bis 12, wobei das Fluoreszenz-markierte Triglycerid Triacylglycerol (TAG) ist.

**14.** Verfahren nach Anspruch 12 oder 13, wobei das Fluoreszenz-markierte Triglycerid jedes Triacylglycerol ist, das mindestens eine NBD enthält.

**15.** Verfahren nach Anspruch 14, wobei das Fluoreszenz-markierte TAG mindestens eine NBD an einer beliebigen Position enthält und Fettsäuren sich an anderen Positionen auf dem TAG befinden.

**16.** Verfahren nach einem der Ansprüche 14 bis 15, wobei das Fluoreszenz-markierte Triacylglycerol 1,2-Dioleoyl-3-NBD-glycerol ist.

**17.** Gemisch zum Messen der Lipidtransferaktivität von MTP nach dem Verfahren nach einem der vorangehenden Ansprüche, wobei das Gemisch umfasst:

- Phosphatidylcholin- (PC-) Akzeptorvesikel,
- Phosphatidylcholin- (PC-) Donorvesikel mit einem darin eingearbeiteten Fluoreszenz-markierten Triglyceridlipid, und
- BSA,

wobei die Konzentration von Phosphatidylcholin der Donorvesikel von 6 $\mu$M bis 18 $\mu$M reicht und die Konzentration von Fluoreszenz-markiertem Triglyceridlipid von 0,21 $\mu$M bis 0,6 $\mu$M reicht, und wobei die Konzentration von Phosphatidylcholin der Akzeptorvesikel von 42 $\mu$M bis 102 $\mu$M reicht, und wobei die Konzentration von BSA 1 mg/ml ist.

**18.** Gemisch nach Anspruch 17, wobei die Konzentration von Phosphatidylcholin der Donorvesikel 13,5 $\mu$M beträgt und die Konzentration von Fluoreszenz-markiertem TAG 0,42 $\mu$M beträgt und/oder wobei die Konzentration von Phosphatidylcholin der Akzeptorvesikel 72 $\mu$M beträgt.

**19.** Gemisch nach Anspruch 18, wobei das Fluoreszenz-markierte Triglycerid Triacylglycerol (TAG) ist.

**20.** Gemisch nach Anspruch 18 oder 19, wobei das Fluoreszenz-markierte Triglycerid jedes Triacylglycerol ist, das mindestens eine NBD enthält.

**21.** Gemisch nach Anspruch 20, wobei das Fluoreszenz-markierte TAG mindestens eine NBD an einer beliebigen Position enthält und Fettsäuren sich an anderen Positionen auf dem TAG befinden.

**22.** Gemisch nach einem der Ansprüche 17 bis 21, wobei das Fluoreszenz-markierte Triacylglycerol 1,2-Dioleoyl-3-NBD-glycerol ist.

**Revendications**

**1.** Procédé de mesure de niveaux de protéine microsomale de transfert des triglycérides (MTP), le procédé comprenant :

(a) la préparation de vésicules donneuses de phosphatidylcholine (PC) à l'intérieur desquelles est incorporé un lipide triglycéride marqué par fluorescence ;
(b) la préparation de vésicules accepteuses de phosphatidylcholine (PC),
(c) l'incubation d'un homogénat cellulaire contenant une MTP ou d'une MTP isolée avec les vésicules accepteuses et les vésicules donneuses marquées pendant une durée et dans des conditions suffisantes pour permettre la liaison du lipide triglycéride marqué par fluorescence à la MTP durant le transfert du lipide marqué des vésicules donneuses aux vésicules accepteuses ; la concentration finale en phosphatidylcholine des vésicules donneuses se trouvant dans la plage de 6 $\mu$M à 18 $\mu$M et la concentration finale en lipide triglycéride marqué par fluorescence se trouvant dans la plage de 0,21 $\mu$M à 0,6 $\mu$M, et la concentration finale en phospholipide phosphatidylcholine des vésicules accepteuses se trouvant dans la plage de 42 $\mu$M à 102 $\mu$M,
(d) la mesure de la fluorescence du lipide triglycéride marqué par fluorescence lié à la MTP, les vésicules étant stabilisées par addition au mélange réactionnel de BSA en une concentration de 1 mg/mL.

2. Procédé selon la revendication 1, dans lequel la concentration finale en phosphatidylcholine des vésicules donneuses est de 13,5 $\mu$M et la concentration finale en lipide triglycéride marqué par fluorescence est de 0,42 $\mu$M, et/ou, dans lequel la concentration finale en phospholipide phosphatidylcholine des vésicules accepteuses est de 72 $\mu$M.

3. Procédé selon la revendication 1 ou 2, dans lequel l'homogénat cellulaire comprend des cellules animales qui expriment la MTP.

4. Procédé selon la revendication 1 ou 2, dans lequel l'homogénat cellulaire comprend des cellules d'insectes ou de microorganismes qui expriment la MTP.

5. Procédé selon la revendication 4, dans lequel le triglycéride marqué par fluorescence est le triacylglycérol (TAG).

6. Procédé selon la revendication 4 ou 5, dans lequel le triglycéride marqué par fluorescence est tout triacylglycérol qui contient au moins un NBD.

7. Procédé selon la revendication 5, dans lequel le TAG marqué par fluorescence contient au moins un NBD à une position quelconque et des acides gras se trouvent à d'autres positions sur le TAG.

8. Procédé selon l'une quelconque des revendications 5 à 7, dans lequel le triacylglycérol marqué par fluorescence est le 1,2-dioléoyl 3-NBD glycérol.

9. Procédé d'identification de composés qui modulent l'activité de transfert des lipides de la MTP, ledit procédé comprenant :

(a) l'incorporation d'un lipide triglycéride marqué par fluorescence dans des vésicules donneuses de phosphatidylcholine (PC) ;
(b) la préparation de vésicules accepteuses de phosphatidylcholine (PC) ;
(c) le mélange d'un aliquote de vésicules accepteuses et des vésicules donneuses marquées avec un composé de test, le composé de test étant un modulateur connu ou inconnu de la MTP ;
(d) l'ajout d'un homogénat cellulaire contenant la MTP ou de MTP isolée au mélange contenant les vésicules donneuses, les vésicules accepteuses, et le composé de test ;
(e) l'incubation d'une première aliquote de vésicules accepteuses, de vésicules donneuses marquées, de composé de test et de MTP pendant une durée et dans des conditions suffisantes pour permettre la liaison du lipide triglycéride marqué par fluorescence à la MTP pendant le transfert du lipide marqué des vésicules donneuses aux vésicules accepteuses ; la concentration en phosphatidylcholine des vésicules donneuses se trouvant dans la plage de 6 $\mu$M à 18 $\mu$M et la concentration du lipide triglycéride marqué par fluorescence se trouvant dans la plage de 0,21 $\mu$M à 0,6 $\mu$M, et la concentration en phospholipide phosphatidylcholine des vésicules accepteuses se trouvant dans la plage de 42 $\mu$M à 102 $\mu$M, les vésicules étant stabilisées par l'ajout de 1 mg/mL de BSA au mélange réactionnel ;
(f) l'incubation d'une deuxième aliquote de vésicules accepteuses, de vésicules donneuses marquées et de MTP pendant une durée et dans des conditions suffisantes pour permettre la liaison du lipide triglycéride marqué par fluorescence à la MTP pendant le transfert du lipide marqué des vésicules donneuses aux vésicules accepteuses ; la concentration en phospholipide phosphatidylcholine des vésicules donneuses se trouvant dans la plage de 6 $\mu$M à 18 $\mu$M et la concentration en lipide triglycéride se trouvant dans la plage de 0,21 $\mu$M à 0,6 $\mu$M, et la concentration en phospholipide phosphatidylcholine des vésicules accepteuses se trouvant dans la plage de 42 $\mu$M à 102 $\mu$M, les vésicules étant stabilisées par l'ajout de 1 mg/mL de BSA au mélange réactionnel ;
(g) la mesure de la fluorescence du lipide triglycéride marqué par fluorescence lié à la MTP obtenu dans les étapes (e) et (f) ; et
(h) la corrélation d'une augmentation de fluorescence des lipides triglycérides marqués par fluorescence liés à la MTP obtenus dans l'étape (e) comparé à la fluorescence du lipide triglycéride marqué par la fluorescence lié à la MTP obtenu dans l'étape (f) à l'identification d'un composé qui entraîne une augmentation de l'activité de transfert des lipides de la MTP, tout en corrélant une diminution de fluorescence du lipide triglycéride marqué par fluorescence lié à la MTP obtenu dans l'étape (e) comparée à la fluorescence du lipide triglycéride marqué par fluorescence lié à la MTP obtenu dans l'étape (f) à l'identification d'un composé qui entraîne une diminution de l'activité de transfert des lipides de la MTP.

10. Procédé selon la revendication 9, dans lequel la concentration finale en phosphatidylcholine des vésicules donneuses est de 13,5 $\mu$M et la concentration en lipide triglycéride marqué par fluorescence est de 0,42 $\mu$M, et/ou, dans lequel la concentration en phospholipide phosphatidylcholine des vésicules accepteuses est de 72 $\mu$M.

11. Procédé selon la revendication 9 ou 10, dans lequel l'homogénat cellulaire comprend des cellules animales qui expriment la MTP.

12. Procédé selon l'une quelconque des revendications 9 à 11, dans lequel l'homogénat cellulaire comprend des cellules d'insectes ou de microorganismes qui expriment la MTP.

13. Procédé de l'une quelconque des revendications 9 à 12, dans lequel le triglycéride marqué par fluorescence est le triacylglycérol (TAG).

14. Procédé selon la revendication 12 ou 13, dans lequel le triglycéride marqué par fluorescence est le triacylglycérol qui contient au moins un NBD.

15. Procédé selon la revendication 14, dans lequel le TAG marqué par fluorescence contient au moins un NBD à une position quelconque et des acides gras se trouvent à d'autres positions sur le TAG.

16. Procédé de l'une quelconque des revendications 14 à 15, dans lequel le triacylglycérol marqué par fluorescence est le 1,2-dioléoyl 3-NBD glycérol.

17. Mélange destiné à mesurer l'activité de transfert des lipides de la MTP selon le procédé de l'une quelconque des revendications précédentes, ledit mélange comprenant :

- des vésicules accepteuses de phosphatidylcholine (PC),
- des vésicules donneuses de phosphatidylcholine (PC) à l'intérieur desquelles est incorporé un triglycéride marqué par fluorescence, et
- de la BSA, la concentration en phosphatidylcholine des vésicules donneuses se trouvant dans la plage de 6 $\mu$M à 18 $\mu$M et la concentration en lipide triglycéride marqué par fluorescence se trouvant dans la plage de 0,21 $\mu$M à 0,6 $\mu$M, et la concentration en phosphatidylcholine des vésicules accepteuses se trouvant dans la plage de 42 $\mu$M à 102 $\mu$M et la concentration en BSA étant de 1 mg/mL.

18. Mélange selon la revendication 17, dans lequel la concentration finale en phosphatidylcholine des vésicules donneuses est de 13,5 $\mu$M et la concentration en TAG marqué par fluorescence est de 0,42 $\mu$M, et/ou, dans lequel la concentration en phospholipide phosphatidylcholine des vésicules accepteuses est de 72 $\mu$M.

19. Mélange selon la revendication 18, dans lequel le triglycéride marqué par fluorescence est le triacylglycérol (TAG).

20. Mélange selon les revendications 18 ou 19, dans lequel le triglycéride marqué par fluorescence est le triacylglycérol qui contient au moins un NBD.

21. Mélange selon la revendication 20, dans lequel le TAG marqué par fluorescence contient au moins un NBD à une position quelconque et des acides gras se trouvent à d'autres positions sur le TAG.

22. Mélange selon l'une quelconque des revendications 17 à 21, dans lequel le triacylglycérol marqué par fluorescence est le 1,2-dioléoyl 3-NBD glycérol.

# FIGURES 1 A-D

# FIGURES 2A-C

# FIGURES 3A-C

# FIGURES 4A-B

# FIGURES 5A-I

# FIGURE 6A

FIGURE 6B

# FIGURES 7A-B

FIGURE 8

NBD-TG

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Hussain, M. M. ; J. Shi ; P. Dreizen.** Microsomal triglyceride transfer protein and its role in apolipoprotein B-lipoprotein assembly. *J. Lipid Res.,* 2003, vol. 44, 22-32
- **Hussain, M. M. ; J. Iqbal ; K. Anwar ; P. Rava ; K. Dai.** Microsomal triglyceride transfer protein: a multifunctional protein. *Front. Biosci.,* 2003, vol. 8, S500-S506
- **Bakillah, A. ; A. El Abbouyi.** The role of microsomal triglyceride transfer protein in lipoprotein assembly: an update. *Front. Biosci.,* 2003, vol. 8, D294-D305
- **Shelness, G. S. ; J. A. Sellers.** Very-low-density lipoprotein assembly and secretion. *Curr. Opin. Lipidol.,* 2001, vol. 12, 151-157
- **Gordon, D. A. ; H. Jamil.** Progress towards understanding the role of microsomal triglyceride transfer protein in apolipo-protein-B lipoprotein assembly. *Biochim. Biophys. Acta,* 2000, vol. 1486, 72-83
- **Wetterau, J. R. ; M. C. M. Lin ; H. Jamil.** Microsomal triglyceride transfer protein. *Biochim. Biophys. Acta,* 1997, vol. 1345, 136-150
- **Hussain, M. M.** A proposed model for the assembly of chylomicrons. *Atherosclerosis,* 2000, vol. 148, 1-15
- **Davis, R. A.** Cell and molecular biology of the assembly and secretion of apolipoprotein B-containing lipoproteins by the liver. *Biochim. Biophys. Acta,* 1999, vol. 1440, 1-31
- **Fisher, E. A. ; H. N. Ginsberg.** Complexity in the secretory pathway: the assembly and secretion of apolipoprotein B-containing lipoproteins. *J. Biol. Chem.,* 2002, vol. 277, 17377-17380
- **Wetterau, J. R. ; L. P. Aggerbeck ; M-E. Bouma ; C. Eisenberg ; A. Munck ; M. Hermier ; J. Schmitz ; G. Gay ; D. J. Rader ; R. E. Gregg.** Absence of microsomal triglyceride transfer protein in individuals with abetalipoproteinemia. *Science,* 1992, vol. 258, 999-1001
- **Shoulders, C. C. ; D. J. Brett ; J. D. Bayliss ; T. M. E. Narcisi ; A. Jarmuz ; T. T. Grantham ; P. R. D. Leoni ; S. Bhattacharya ; R. J. Pease ; P. M. Cullen.** Abetalipoproteinemia is caused by defects of the gene encoding the 97 kDa subunit of a microsomal triglyceride transfer protein. *Hum. Mol. Genet.,* 1993, vol. 2, 2109-2116
- **Jamil, H. ; D. A. Gordon ; D. C. Eustice ; C. M. Brooks ; J. K. Dickson, Jr. ; Y. Chen ; B. Ricci ; C-H. Chu ; T. W. Harrity ; C. P. Ciosek, Jr.** An inhibitor of the microsomal triglyceride transfer protein inhibits apoB secretion from HepG2 cells. *Proc. Natl. Acad. Sci. USA,* 1996, vol. 93, 11991-11995
- **Haghpassand, M. ; D. Wilder ; J. B. Moberly.** Inhibition of apolipoprotein B and triglyceride secretion in human hepatoma cells (HepG2). *J. Lipid Res.,* 1996, vol. 37, 1468-1480
- **Benoist, F. ; T. Grand-Perret.** Co-translational degradation of apolipoprotein B100 by the proteasome is prevented by microsomal triglyceride transfer protein-synchronized translation studies on HepG2 cells treated with an inhibitor of microsomal triglyceride transfer protein. *J. Biol. Chem.,* 1997, vol. 272, 20435-20442
- **Gordon, D. A. ; H. Jamil ; D. Sharp ; D. Mullaney ; Z. Yao ; R. E. Gregg ; J. Wetterau.** Secretion of apolipoprotein B-containing lipoproteins from HeLa cells is dependent on expression of the microsomal triglyceride transfer protein and is regulated by lipid availability. *Proc. Natl. Acad. Sci. USA.,* 1994, vol. 91, 7628-7632
- **Leiper, J. M. ; J. D. Bayliss ; R. J. Pease ; D. J. Brett ; J. Scott ; C. C. Shoulders.** Microsomal triglyceride transfer protein, the abetalipoproteinemia gene product, mediates the secretion of apolipoprotein B-containing lipoproteins from heterologous cells. *J. Biol. Chem.,* 1994, vol. 269, 21951-21954
- **Raabe, M. ; M. M. Véniant ; M. A. Sullivan ; C. H. Zlot ; J. Björkegren ; L. B. Nielsen ; J. S. Wong ; R. L. Hamilton ; S. G. Young.** Analysis of the role of microsomal triglyceride transfer protein in the liver of tissue-specific knockout mice. *J. Clin. Invest.,* 1999, vol. 103, 1287-1298
- **Wang, Y. W. ; K. Tran ; Z. M. Yao.** The activity of microsomal triglyceride transfer protein is essential for accumulation of triglyceride within microsomes in McA-RH7777 cells-a unified model for the assembly of very low density lipoproteins. *J. Biol. Chem.,* 1999, vol. 274, 27793-27800
- **Kulinski, A. ; S. Rustaeus ; J. E. Vance.** Microsomal triacylglycerol transfer protein is required for lumenal accretion of triacylglycerol not associated with Apo B, as well as for Apo B lipidation. *J. Biol. Chem.,* 2002, vol. 277, 31516-31525

- **Wetterau, J. R. ; D. B. Zilversmit.** A triglyceride and cholesteryl ester transfer protein associated with liver microsomes. *J. Biol. Chem.,* 1984, vol. 259, 10863-10866
- **Wetterau, J. R. ; D. B. Zilversmit.** Purification and characterization of microsomal triglyceride and cholesteryl ester transfer protein from bovine liver microsomes. *Chem. Phys. Lipids,* 1985, vol. 38, 205-222
- **Jamil, H. ; J. K. Dickson, Jr. ; C-H. Chu ; M. W. Lago ; J. K. Rinehart ; S. A. Biller ; R. E. Gregg ; J. R. Wetterau.** Microsomal triglyceride transfer protein. Specificity of lipid binding and transport. *J. Biol. Chem.,* 1995, vol. 270, 6549-6554
- **Chang, G. ; R. B. Ruggeri ; H. J. Harwood, Jr.** Microsomal triglyceride transfer protein (MTP) inhibitors: discovery of clinically active inhibitors using high-throughput screening and parallel synthesis paradigms. *Curr. Opin. Drug Discov. Dev.,* 2002, vol. 5, 562-570
- **Hussain, M. M. ; A. Bakillah ; H. Jamil.** Apolipoprotein B binding to microsomal triglyceride transfer protein decreases with increases in length and lipidation: implications in lipoprotein bio-synthesis. *Biochemistry,* 1997, vol. 36, 13060-13067
- **Hussain, M. M. ; A. Bakillah ; N. Nayak ; G. S. Shelness.** Amino acids 430-570 in apolipoprotein B are critical for its binding to microsomal triglyceride transfer protein. *J. Biol. Chem.,* 1998, vol. 273, 25612-25615
- **Bakillah, A. ; H. Jamil ; M. M. Hussain.** Lysine and arginine residues in the N-terminal 18% of apolipoprotein B are critical for its binding to microsomal triglyceride transfer protein. *Biochemistry,* 1998, vol. 37, 3727-3734
- **Bakillah, A. ; N. Nayak ; U. Saxena ; R. M. Medford ; M. M. Hussain.** Decreased secretion of apoB follows inhibition of apoB-MTP binding by a novel antagonist. *Biochemistry,* 2000, vol. 39, 4892-4899
- **Bakillah, A. ; M. M. Hussain.** Binding of microsomal triglyceride transfer protein to lipids results in increased affinity for apolipoprotein B: Evidence for stable microsomal MTP-lipid complexes. *J. Biol. Chem.,* 2001, vol. 276, 31466-31473
- **Tietge, U. J. F. ; A. Bakillah ; C. Maugeais ; K. Tsukamoto ; M. M. Hussain ; D. J. Rader.** Hepatic overexpression of microsomal triglyceride transfer protein (MTP) results in increased in vivo secretion of VLDL triglycerides and apolipoprotein B. *J. Lipid Res.,* 1999, vol. 40, 2134-2139
- **Wetterau, J. R. ; L. P. Aggerbeck ; P. M. Laplaud ; L. R. McLean.** Structural properties of the microsomal triglyceride-transfer protein complex. *Biochemistry,* 1991, vol. 30, 4406-4412
- **Atzel, A. ; J. R. Wetterau.** Mechanism of microsomal triglyceride transfer protein catalyzed lipid transport. *Biochemistry,* 1993, vol. 32, 10444-10450
- **Atzel, A. ; J. R. Wetterau.** Identification of two classes of lipid molecule binding sites on the microsomal triglyceride transfer protein. *Biochemistry,* 1994, vol. 33, 15382-15388
- **Bradford, M. M.** A rapid and sensitive method for the quantitation of microgram quantities of protein utilizing the principle of protein-dye binding. *Anal. Biochem,* 1976, vol. 72, 248-254
- **Chang, T. Y. ; J. S. Limanek ; C. C. Chang.** A simple and efficient procedure for the rapid homogenization of cultured animal cells grown in monolayer. *Anal. Biochem,* 1981, vol. 116, 298-302